# EUROPEAN PATENT APPLICATION

(11) **EP 4 386 007 A1**
(43) Date of publication of application: **19.06.2024**
(21) Application number: 22855548.8
(22) Date of filing: 12.08.2022
(51) Int. Cl.: C07K 16/46, C07K 19/00, C12N 15/62, C12N 15/63, C12N 5/10, A61K 47/68, A61K 38/17, A61K 38/21, A61P 35/00, A61P 31/00, A61P 37/06

(54) **BISPECIFIC RECOMBINANT PROTEIN AND USE THEREOF**

(30) Priority: 12.08.2021 CN 202110926743
(71) Applicant: Shanghai TTM-BIO Technology Co., Ltd., Shanghai 201304 (CN)
(72) Inventor: HE, Ke, Shanghai 201318 (CN); SONG, Liping, Shanghai 201318 (CN); FAN, Yi, Shanghai 201318 (CN); CHEN, Yingjiao, Shanghai 201318 (CN)
(74) Representative: Dehns
(86) International application number: PCT/CN2022/112300
(87) International publication number: WO 2023/016568

(57) **Abstract**

Disclosed are a bispecific recombinant protein and a use thereof. The bispecific recombinant protein comprises a first functional binding region, a second functional binding region and an Fc region; the first functional binding region comprises a first chain and a second chain, and a C-terminus of the first chain and a C-terminus of the second functional binding region are separately connected to an N-terminus of the Fc region directly or by means of a linker sequence; and a C-terminus of the second chain is connected to an N-terminus of the second functional binding region directly or by means of a linker sequence. Further disclosed is an application of the recombinant protein in terms of anti-tumor, anti-virus, liver disease treatment, and other applications. The recombinant protein may significantly improve the killing efficacy of target cells while significantly reducing toxic side effects caused by binding to non-target cells, and has good safety and good freeze-thaw stability.

## Description

The present application claims the priority of Chinese patent application 2021109267431 filed on August 12, 2021. The contents of the above Chinese patent application are incorporated herein by reference in its entirety.

### TECHNICAL FIELD

The present disclosure belongs to the field of biomedicine, and specifically relates to a bispecific recombinant protein and use thereof.

### BACKGROUND

Interferons are a group of active proteins with various functions, which are cytokines produced mainly by monocytes and lymphocytes, and are classified into three types, including type I, type II, and type III. Type I interferons include IFNα and IFNβ, and IFNα may also include subtypes, for example, IFNα2 and IFNα4. Type II and III interferons may include IPNγ, IPNλ1 (IL-29), IFNλ2 (IL-28a), and IFNλ3 (IL-28b). Interferons have various biological activities, for example, broad-spectrum anti-virus, influence on cell growth and differentiation, and regulation of immune functions, etc. Among them, interferon α (IFNα) has a wide range of antiviral and anti-tumor effects. For example, it activates effector cells via binding to IFNα receptors, enhances the activity of immune cells, for example natural killer cells, etc., inhibits the proliferation of viruses and tumor cells, and induces tumor cell apoptosis, etc. Therefore, it is one of the biological products that are widely used in clinical practice, mainly for the treatment of liver diseases (e.g., chronic hepatitis B and hepatitis C), viral diseases (e.g., viral respiratory diseases, skin diseases, hematological diseases, and gynecological diseases), and specific tumors (e.g., chronic myelogenous leukemia, melanoma, lymphoma, renal cancer, etc.), and serves as adjuvant therapy for tumors and malignant hematological diseases.

However, since IFNα receptors are widely expressed in various tissues and organs of the human body, the clinical use of IFNα often leads to toxic side effects, for example, fever, fatigue, myalgia, hepatotoxicity, myelosuppression, and neurotoxicity, etc. Extensive clinical research data show that PEG-IFNα (6 µg/kg/week for 8 weeks in treatment; 3 µg/kg/week in maintenance) can cause grade 3 or higher severe adverse events (SAEs) in more than 60% of patients during the adjuvant therapy for melanoma (Practical Guidelines for the Management of Interferon-α-2b Side Effects in Patients Receiving Adjuvant Treatment for Melanoma. Cancer 2008; 112: 982-94), and the maximum tolerated dose (MTD) of PEG-IFNα for the treatment of chronic myeloid leukemia (CML) in clinical trials ranges from 7.5 to 9 µg/kg (Long-Term Results of the Randomized Phase III Trial EORTC 18991 of Adjuvant Therapy With Pegylated Interferon Alfa-2b Versus Observation in Resected Stage III Melanoma. JOURNAL OF CLINICAL ONCOLOGY, VOLUME 30, NUMBER 31, NOVEMBER 1, 2012). However, based on preclinical *in vivo* and *in vitro* efficacy data, the optimal tumor-suppression dose needs to be at least 100 times higher than the tolerated dose in clinical trials (Treating Cancer with PEG Intron *Pharmacokinetic Profile and Dosing Guidelines for an Improved Interferon-Alpha-2b Formulation.* TALPAZ et al BLOOD, 15 SEPTEMBER 2001. VOLUME 98, NUMBER 6).

In addition, interferons generally have poor freeze-thaw stability due to their product characteristics, for example, the polymer content gradually increases after repeated freeze-thaw of the recombinant human albumin interferon-α2b fusion protein (rHSA-IFN-α2b). After 4 freeze-thaw cycles, the polymer content reaches 17.91%, and thus the recombinant human albumin interferon-α2b fusion protein should not be freeze-thawed (Yi Xia et al., The Stability Study of Recombinant Human Albumin Interferon α-2b Fusion Protein, Journal of Biology, 2008, 25(006): 38-40), resulting in more restrictions on production, transportation, and use.

Therefore, there is an urgent need to invent an antibody-cytokine fusion protein (i.e., bispecific recombinant protein) with high efficiency and low toxicity, which can significantly increase the therapeutic dose of IFNα and further improve the effects of anti-tumor, anti-virus, and treatment of liver disease on the premise of human tolerance. At the same time, the bispecific recombinant protein should have better freeze-thaw stability than interferon.

### CONTENT OF THE PRESENT INVENTION

The technical problem to be solved by the present disclosure is to provide a bispecific recombinant protein and use thereof in order to overcome the poor efficacy of monoclonal antibody and the deficiencies in safety and freeze-thaw stability of interferon α in the prior art. The bispecific recombinant protein of the present disclosure has the effect of directional regulation of immunity, which can significantly improve the targeting of the recombinant protein to a target, and can also significantly reduce the toxic side effects caused by the non-target proteins targeting non-target organs, tissues, and cells generated during the preparation process.

The present disclosure solves the above technical problem through the following technical solutions.

The first aspect of the present disclosure provides a bispecific recombinant protein, including a first functional binding region, a second functional binding region, and an Fc region; the first functional binding region includes a first chain and a second chain, and C-terminus of the first chain and C-terminus of the second functional binding region are separately linked to N-terminus of the Fc region directly or via a linker sequence; C-terminus of the second chain is linked to N-terminus of the second functional binding region directly or via a linker sequence;
the first functional binding region includes a heavy chain variable region (VH), a light chain variable region (VL), a heavy chain constant region 1 (CH1), and a light chain constant region (CL);
the second functional binding region comprises an interferon, a truncated variant thereof, or a mutant thereof.

In some embodiments of the present disclosure, C-terminus of the CL or CH1 is linked to N-terminus of the Fc region directly or via a linker sequence; C-terminus of the corresponding CH1 or CL is linked to N-terminus of the second functional binding region directly or via a linker sequence.

In some embodiments of the present disclosure, the first chain includes VH and CH1, VH and CL, VL and CH1, or VL and CL; the second chain includes VH and CH1, VH and CL, VL and CH1, or VL and CL. The first chain is not identical to the second chain, and a combination of the first chain and the second chain includes four elements: VH, CH1, VL, and CL. For example, when the first chain includes VH and CH1, the second chain includes VL and CL; when the first chain includes VH and CL, the second chain includes VL and CH1.

In some embodiments of the present disclosure, the interferon is type I interferon, for example IFNα.

In some preferred embodiments of the present disclosure, the IFNα is selected from one or more of IFN-α1a, IFN-α1b, IFN-α2a, IFN-α2b, IFN-α4a, IFN-α4b, IFN-α5, IFN-α6, IFN-α7, IFN-α8, IFN-α10, IFN-α14, IFN-α16, IFN-α17, and IFN-α21.

In some embodiments of the present disclosure, the interferon is IFN-α2b.

In some embodiments of the present disclosure, the second functional binding region comprises an amino acid sequence selected from any one of the following a1) and a2): a1) SEQ ID NO: 17; a2) an amino acid sequence obtained by addition, deletion, modification, and/or substitution of at least one amino acid residue of the amino acid sequence as shown in SEQ ID NO: 17, whose monomer has binding affinity to IFNα receptor and the binding affinity is no higher than that of an a1) monomer to IFNα receptor.

In some preferred embodiments of the present disclosure, amino acid sequence of a2) monomer is the amino acid sequence obtained by substitution at a corresponding site of the amino acid sequence as shown in SEQ ID NO: 17; the substitution at the corresponding site is selected from one or more of L26A, L30A, A145G, R149A, and S152A.

In some specific embodiments of the present disclosure, the a2) monomer has a single-site substitution of L26A, L30A, A145G, R149A, or S152A, and the corresponding amino acid sequences are shown in SEQ ID NOs: 18 to 22, respectively.

In some embodiments of the present disclosure, the VH is linked to CH1 or CL directly or via a linker sequence; the VL is linked to CL or CH1 directly or via a linker sequence.

In some specific embodiments of the present disclosure, when VL is linked to CL, VH is linked to CH1; when VL is linked to CH1, VH is linked to CL.

In some embodiments of the present disclosure, the first functional binding region targets a tumor cell or an immune cell.

Specifically, the first functional binding region targets any one or more of the following targets: GPC3, BCMA, PD-L1, Trop-2, 5T4, AGS-16, ALK1, ANG-2, B7-H3, B7-H4, c-fms, c-Met, CA6, CD123, CD19, CD20, CD22, CD24, EpCAM, CD30, CD32b, CD37, CD38, CD40, CD52, CD70, CD74, CD79b, CD98, CEA, CEACAM5, CLDN18.2, CLDN6, CS1, CXCR4, DLL-4, EGFR, EGFRvIII, EGP-1, ENPP3, EphA3, ETBR, FGFR2, FN, FR-α, GCC, GD2, GPNMB, HER2, HER3, HLA-DR, ICAM-1, IGF-1R, IL-3R, LIV-1, MSLN, MUC16, MUC1, NaPi2b, Nectin-4, Notch 2, Notch 1, PD-L2, PDGFR-α, PS, PSMA, SLTRK6, STEAP1, TEM1, VEGFR, CD25, CD27L, DKK-1, CSF-1R, MSB0010718C, CD138, Siglec15, and CD155.

In some embodiments of the present disclosure, the first functional binding region targets GPC3; the first functional binding region comprises an antigen-binding fragment of a specific anti-GPC3 monoclonal antibody.

In some specific embodiments of the present disclosure, heavy chain variable region (VH) sequence of the specific anti-GPC3 monoclonal antibody is shown in SEQ ID NO: 1 and light chain variable region (VL) sequence is shown in SEQ ID NO: 2.

In other embodiments of the present disclosure, the first functional binding region targets PD-L1; the first functional binding region comprises an antigen-binding fragment of a specific anti-PD-L1 monoclonal antibody.

In some specific embodiments of the present disclosure, heavy chain variable region (VH) sequence of the specific anti-PD-L1 monoclonal antibody is shown in SEQ ID NO: 5 and light chain variable region (VL) sequence is shown in SEQ ID NO: 6.

In other embodiments of the present disclosure, the first functional binding region targets BCMA; the first functional binding region comprises an antigen-binding fragment of a specific anti-BCMA monoclonal antibody.

In some specific embodiments of the present disclosure, heavy chain variable region (VH) sequence of the specific anti-BCMA monoclonal antibody is shown in SEQ ID NO: 7 or SEQ ID NO: 9 and corresponding light chain variable region (VL) sequence is shown in SEQ ID NO: 8 or SEQ ID NO: 10.

In other embodiments of the present disclosure, the first functional binding region targets Trop-2; the first functional binding region comprises an antigen-binding fragment of a specific anti-Trop-2 monoclonal antibody.

In some specific embodiments of the present disclosure, heavy chain variable region (VH) sequence of the specific anti-Trop-2 monoclonal antibody is shown in SEQ ID NO: 11 and light chain variable region (VL) sequence is shown in SEQ ID NO: 12.

In some embodiments of the present disclosure, the linker sequence has a length of no more than 20 amino acid residues, and the linker sequence is selected from the group consisting of (GGGGS)n, (GGGS)n, (GGS)n, (G)n, (GS)n, (EAAAK)n, and (XP)n, wherein n is a natural number; for example, amino acid sequence of the linker sequence is shown in SEQ ID NOs: 13 to 16.

In some embodiments of the present disclosure, the bispecific recombinant protein consists of a chain A and a chain B; wherein, structure of the chain A is VH-CH1-FcA region, and structure of the chain B is VL-CL-second functional binding region-FcB region.

In other embodiments of the present disclosure, structure of the chain A is VH-CL-FcA region, and structure of the chain B is VL-CH1-second functional binding region-FcB region.

In other embodiments of the present disclosure, structure of the chain A is VL-CH1-FcA region, and structure of the chain B is VH-CL-second functional binding region-FcB region.

In other embodiments of the present disclosure, structure of the chain A is VL-CL-FcA region, and structure of the chain B is VH-CH1-second functional binding region-FcB region.

In the present disclosure, the chain A and the chain B are combined via one or more bindings selected from intermolecular forces, covalent bonds, and salt bonds.

The second aspect of the present disclosure provides a bispecific recombinant protein, including a first functional binding region, a second functional binding region, and an Fc region; the first functional binding region includes a heavy chain variable region (VH), a light chain variable region (VL), a heavy chain constant region 1 (CH1), and a light chain constant region (CL); the second functional binding region comprises an interferon, a truncated variant thereof, or a mutant thereof; the bispecific recombinant protein consists of a first arm and a second arm, and the first arm consists of a chain 1 and a chain 2; wherein structure of the chain 1 is VH-CH1-FcA region, and structure of the chain 2 is VL-CL; or, structure of the chain 1 is VH-CL-FcA region, and structure of the chain 2 is VL-CH1; or, structure of the chain 1 is VL-CH1-FcA region, and structure of the chain 2 is VH-CL; or, structure of the chain 1 is VL-CL-FcA region, and structure of the chain 2 is VH-CH1; structure of the second arm is second functional binding region-FcB region, and the FcA region and the FcB region constitute the Fc region of the bispecific recombinant protein; in the second arm, amino acid sequence of the second functional binding region comprises any one of SEQ ID NOs: 17 to 22.

When the first functional binding region targets GPC3, the first functional binding region comprises an antigen-binding fragment of a specific anti-GPC3 monoclonal antibody; when the first functional binding region targets PD-L1, the first functional binding region comprises an antigen-binding fragment of a specific anti-PD-L1 monoclonal antibody; when the first functional binding region targets BCMA, the first functional binding region comprises an antigen-binding fragment of a specific anti-BCMA monoclonal antibody; when the first functional binding region targets Trop-2, the first functional binding region comprises an antigen-binding fragment of a specific anti-Trop-2 monoclonal antibody.

In some embodiments of the present disclosure, when the first functional binding region targets GPC3, in the first arm, amino acid sequence of VH comprises SEQ ID NO: 1, amino acid sequence of CH1 comprises SEQ ID NO: 31, amino acid sequence of VL comprises SEQ ID NO: 2, and amino acid sequence of CL comprises SEQ ID NO: 32.

In some embodiments of the present disclosure, when the first functional binding region targets PD-L1, in the first arm, amino acid sequence of VH comprises SEQ ID NO: 5, amino acid sequence of CH1 comprises SEQ ID NO: 31, amino acid sequence of VL comprises SEQ ID NO: 6, and amino acid sequence of CL comprises SEQ ID NO: 32.

In some embodiments of the present disclosure, when the first functional binding region targets BCMA, in the first arm, amino acid sequence of VH comprises SEQ ID NO: 7 or 9, amino acid sequence of CH1 comprises SEQ ID NO: 31, amino acid sequence of VL comprises SEQ ID NO: 8 or 10, and amino acid sequence of CL comprises SEQ ID NO: 32.

In some embodiments of the present disclosure, when the first functional binding region targets Trop-2, in the first arm, amino acid sequence of VH comprises SEQ ID NO: 11, amino acid sequence of CH1 comprises SEQ ID NO: 31, amino acid sequence of VL comprises SEQ ID NO: 12, and amino acid sequence of CL comprises SEQ ID NO: 32.

In the present disclosure, the Fc region of the bispecific recombinant protein is Fc region derived from IgG1, IgG2, IgG3, or IgG4; the Fc region comprises a natural sequence or a non-natural sequence.

In some embodiments of the present disclosure, the Fc region is Fc region derived from human IgG1, IgG2, IgG3, or IgG4.

In some preferred embodiments of the present disclosure, the FcA region and the FcB region are heterodimerized through knobs-into-holes.

In some specific embodiments of the present disclosure, when amino acid sequence of the FcA region comprises SEQ ID NO: 25, amino acid sequence of the FcB region comprises SEQ ID NO: 25.

In other specific embodiments of the present disclosure, when amino acid sequence of the FcA region comprises SEQ ID NO: 26, amino acid sequence of the FcB region comprises SEQ ID NO: 27.

In other specific embodiments of the present disclosure, when amino acid sequence of the FcA region comprises SEQ ID NO: 27, amino acid sequence of the FcB region comprises SEQ ID NO: 26.

In other specific embodiments of the present disclosure, when amino acid sequence of the FcA region comprises SEQ ID NO: 28, amino acid sequence of the FcB region comprises SEQ ID NO: 28.

In other specific embodiments of the present disclosure, when amino acid sequence of the FcA region comprises SEQ ID NO: 29, amino acid sequence of the FcB region comprises SEQ ID NO: 30.

In other specific embodiments of the present disclosure, when amino acid sequence of the FcA region comprises SEQ ID NO: 30, amino acid sequence of the FcB region comprises SEQ ID NO: 29.

In some embodiments of the present disclosure, the first functional binding region targets GPC3; in the chain A, amino acid sequence of VH comprises SEQ ID NO: 1, amino acid sequence of VL comprises SEQ ID NO: 2, amino acid sequence of CH1 comprises SEQ ID NO: 31, amino acid sequence of CL comprises SEQ ID NO: 32, and amino acid sequence of the FcA region comprises SEQ ID NO: 26 or SEQ ID NO: 27;
in the chain B, amino acid sequence of VH comprises SEQ ID NO: 1, amino acid sequence of VL comprises SEQ ID NO: 2, amino acid sequence of CH1 comprises SEQ ID NO: 31, amino acid sequence of CL comprises SEQ ID NO: 32, amino acid sequence of the second functional binding region comprises SEQ ID NO: 17, 18, 19, 21, or 22, and amino acid sequence of the FcB region comprises SEQ ID NO: 27 or SEQ ID NO: 26.

In some specific embodiments of the present disclosure, among the amino acid sequences of the chain B, C-terminus of SEQ ID NO: 2 or SEQ ID NO: 1 is linked to N-terminus of SEQ ID NO: 32 directly, C-terminus of SEQ ID NO: 32 is linked to N-terminus of SEQ ID NO: 17, SEQ ID NO: 18, SEQ ID NO: 19, SEQ ID NO: 21, or SEQ ID NO: 22 via a linker sequence, and the linker sequence is selected from any one of SEQ ID NOs: 13 to 16.

In some specific embodiments of the present disclosure, when amino acid sequence of the chain A consists of sequentially linked SEQ ID NO: 1, 31, and the FcA region,
amino acid sequence of the chain B consists of sequentially linked SEQ ID NO: 2, 32, 13, 17, and the FcB region;
or, amino acid sequence of the chain B consists of sequentially linked SEQ ID NO: 2, 32, 13, 18, and the FcB region;
or, amino acid sequence of the chain B consists of sequentially linked SEQ ID NO: 2, 32, 13, 19, and the FcB region;
or, amino acid sequence of the chain B consists of sequentially linked SEQ ID NO: 2, 32, 13, 20, and the FcB region;
or, amino acid sequence of the chain B consists of sequentially linked SEQ ID NO: 2, 32, 13, 21, and the FcB region;
or, amino acid sequence of the chain B consists of sequentially linked SEQ ID NO: 2, 32, 13, 22, and the FcB region;
or, amino acid sequence of the chain B consists of sequentially linked SEQ ID NO: 2, 32, 14, 17, and the FcB region;
or, amino acid sequence of the chain B consists of sequentially linked SEQ ID NO: 2, 32, 14, 18, and the FcB region;
or, amino acid sequence of the chain B consists of sequentially linked SEQ ID NO: 2, 32, 14, 19, and the FcB region;
or, amino acid sequence of the chain B consists of sequentially linked SEQ ID NO: 2, 32, 14, 20, and the FcB region;
or, amino acid sequence of the chain B consists of sequentially linked SEQ ID NO: 2, 32, 14, 21, and the FcB region;
or, amino acid sequence of the chain B consists of sequentially linked SEQ ID NO: 2, 32, 14, 22, and the FcB region;
or, amino acid sequence of the chain B consists of sequentially linked SEQ ID NO: 2, 32, 15, 17, and the FcB region;
or, amino acid sequence of the chain B consists of sequentially linked SEQ ID NO: 2, 32, 15, 18, and the FcB region;
or, amino acid sequence of the chain B consists of sequentially linked SEQ ID NO: 2, 32, 15, 19, and the FcB region;
or, amino acid sequence of the chain B consists of sequentially linked SEQ ID NO: 2, 32, 15, 20, and the FcB region;
or, amino acid sequence of the chain B consists of sequentially linked SEQ ID NO: 2, 32, 15, 21, and the FcB region;
or, amino acid sequence of the chain B consists of sequentially linked SEQ ID NO: 2, 32, 15, 22, and the FcB region;
or, amino acid sequence of the chain B consists of sequentially linked SEQ ID NO: 2, 32, 16, 17, and the FcB region;
or, amino acid sequence of the chain B consists of sequentially linked SEQ ID NO: 2, 32, 16, 18, and the FcB region;
or, amino acid sequence of the chain B consists of sequentially linked SEQ ID NO: 2, 32, 16, 19, and the FcB region;
or, amino acid sequence of the chain B consists of sequentially linked SEQ ID NO: 2, 32, 16, 21, and the FcB region;
or, amino acid sequence of the chain B consists of sequentially linked SEQ ID NO: 2, 32, 16, 22, and the FcB region.

In the above embodiment, when amino acid sequence of the FcA region is SEQ ID NO: 26, amino acid sequence of the FcB region is SEQ ID NO: 27. When amino acid sequence of the FcA region is SEQ ID NO: 27, amino acid sequence of the FcB region is SEQ ID NO: 26.

In some embodiments of the present disclosure, the first functional binding region targets PD-L1; in the chain A, amino acid sequence of VH comprises SEQ ID NO: 5, amino acid sequence of VL comprises SEQ ID NO: 6, amino acid sequence of CH1 comprises SEQ ID NO: 31, amino acid sequence of CL comprises SEQ ID NO: 32, and amino acid sequence of the FcA region comprises SEQ ID NO: 26, SEQ ID NO: 27, SEQ ID NO: 29, or SEQ ID NO: 30;
in the chain B, amino acid sequence of VH comprises SEQ ID NO: 5, amino acid sequence of VL comprises SEQ ID NO: 6, amino acid sequence of CH1 comprises SEQ ID NO: 31, amino acid sequence of CL comprises SEQ ID NO: 32, amino acid sequence of the second functional binding region comprises any one of SEQ ID NOs: 17 to 22, and amino acid sequence of the FcB region comprises SEQ ID NO: 27, SEQ ID NO: 26, SEQ ID NO: 30, or SEQ ID NO: 29.

In some specific embodiments of the present disclosure, among the amino acid sequences of the chain B, C-terminus of SEQ ID NO: 6 or SEQ ID NO: 5 is linked to N-terminus of SEQ ID NO: 32 directly, C-terminus of SEQ ID NO: 32 is linked to N-terminus of any one of SEQ ID NOs: 17 to 22 via a linker sequence, and the linker sequence is selected from any one of SEQ ID NOs: 13 to 16.

In some embodiments of the present disclosure, when amino acid sequence of the chain A consists of sequentially linked SEQ ID NO: 5, 31, and the FcA region,
amino acid sequence of the chain B consists of sequentially linked SEQ ID NO: 6, 32, 13, 17, and the FcB region;
or, amino acid sequence of the chain B consists of sequentially linked SEQ ID NO: 6, 32, 13, 18, and the FcB region;
or, amino acid sequence of the chain B consists of sequentially linked SEQ ID NO: 6, 32, 13, 19, and the FcB region;
or, amino acid sequence of the chain B consists of sequentially linked SEQ ID NO: 6, 32, 13, 20, and the FcB region;
or, amino acid sequence of the chain B consists of sequentially linked SEQ ID NO: 6, 32, 13, 21, and the FcB region;
or, amino acid sequence of the chain B consists of sequentially linked SEQ ID NO: 6, 32, 13, 22, and the FcB region;
or, amino acid sequence of the chain B consists of sequentially linked SEQ ID NO: 6, 32, 14, 17, and the FcB region;
or, amino acid sequence of the chain B consists of sequentially linked SEQ ID NO: 6, 32, 14, 18, and the FcB region;
or, amino acid sequence of the chain B consists of sequentially linked SEQ ID NO: 6, 32, 14, 19, and the FcB region;
or, amino acid sequence of the chain B consists of sequentially linked SEQ ID NO: 6, 32, 14, 20, and the FcB region;
or, amino acid sequence of the chain B consists of sequentially linked SEQ ID NO: 6, 32, 14, 21, and the FcB region;
or, amino acid sequence of the chain B consists of sequentially linked SEQ ID NO: 6, 32, 14, 22, and the FcB region;
or, amino acid sequence of the chain B consists of sequentially linked SEQ ID NO: 6, 32, 15, 17, and the FcB region;
or, amino acid sequence of the chain B consists of sequentially linked SEQ ID NO: 6, 32, 15, 18, and the FcB region;
or, amino acid sequence of the chain B consists of sequentially linked SEQ ID NO: 6, 32, 15, 19, and the FcB region;
or, amino acid sequence of the chain B consists of sequentially linked SEQ ID NO: 6, 32, 15, 20, and the FcB region;
or, amino acid sequence of the chain B consists of sequentially linked SEQ ID NO: 6, 32, 15, 21, and the FcB region;
or, amino acid sequence of the chain B consists of sequentially linked SEQ ID NO: 6, 32, 15, 22, and the FcB region;
or, amino acid sequence of the chain B consists of sequentially linked SEQ ID NO: 6, 32, 16, 17, and the FcB region;
or, amino acid sequence of the chain B consists of sequentially linked SEQ ID NO: 6, 32, 16, 18, and the FcB region;
or, amino acid sequence of the chain B consists of sequentially linked SEQ ID NO: 6, 32, 16, 19, and the FcB region;
or, amino acid sequence of the chain B consists of sequentially linked SEQ ID NO: 6, 32, 16, 20, and the FcB region;
or, amino acid sequence of the chain B consists of sequentially linked SEQ ID NO: 6, 32, 16, 21, and the FcB region;
or, amino acid sequence of the chain B consists of sequentially linked SEQ ID NO: 6, 32, 16, 22, and the FcB region.

In the above embodiment, when amino acid sequence of the FcA region is SEQ ID NO: 26, amino acid sequence of the FcB region is SEQ ID NO: 27. When amino acid sequence of the FcA region is SEQ ID NO: 27, amino acid sequence of the FcB region is SEQ ID NO: 26. When amino acid sequence of the FcA region is SEQ ID NO: 29, amino acid sequence of the FcB region is SEQ ID NO: 30. When amino acid sequence of the FcA region is SEQ ID NO: 30, amino acid sequence of the FcB region is SEQ ID NO: 29.

In some embodiments of the present disclosure, the first functional binding region targets BCMA.

In some preferred embodiments of the present disclosure, in the chain A, amino acid sequence of VH comprises SEQ ID NO: 7 or 9, amino acid sequence of VL comprises SEQ ID NO: 8 or 10, amino acid sequence of CH1 comprises SEQ ID NO: 31, amino acid sequence of CL comprises SEQ ID NO: 32, and amino acid sequence of the FcA region comprises SEQ ID NO: 26 or SEQ ID NO: 27;
in the chain B, amino acid sequence of VH comprises SEQ ID NO: 7 or 9, amino acid sequence of VL comprises SEQ ID NO: 8 or 10, amino acid sequence of CH1 comprises SEQ ID NO: 31, amino acid sequence of CL comprises SEQ ID NO: 32, amino acid sequence of the second functional binding region comprises any one of SEQ ID NOs: 17 to 22, and amino acid sequence of the FcB region comprises SEQ ID NO: 27 or SEQ ID NO: 26.

In some specific embodiments of the present disclosure, among the amino acid sequences of the chain B, C-terminus of SEQ ID NO: 7, 8, 9, or 10 is linked to N-terminus of SEQ ID NO: 32 directly, C-terminus of SEQ ID NO: 32 is linked to N-terminus of any one of SEQ ID NOs: 17 to 22 via a linker sequence, and the linker sequence is selected from any one of SEQ ID NOs: 13 to 16.

In some embodiments of the present disclosure, when amino acid sequence of the chain A consists of sequentially linked SEQ ID NO: 7, 31, and the FcA region,
amino acid sequence of the chain B consists of sequentially linked SEQ ID NO: 8, 32, 13, 17, and the FcB region;
or, amino acid sequence of the chain B consists of sequentially linked SEQ ID NO: 8, 32, 13, 18, and the FcB region;
or, amino acid sequence of the chain B consists of sequentially linked SEQ ID NO: 8, 32, 13, 19, and the FcB region;
or, amino acid sequence of the chain B consists of sequentially linked SEQ ID NO: 8, 32, 13, 20, and the FcB region;
or, amino acid sequence of the chain B consists of sequentially linked SEQ ID NO: 8, 32, 13, 21, and the FcB region;
or, amino acid sequence of the chain B consists of sequentially linked SEQ ID NO: 8, 32, 13, 22, and the FcB region;
or, amino acid sequence of the chain B consists of sequentially linked SEQ ID NO: 8, 32, 14, 17, and the FcB region;
or, amino acid sequence of the chain B consists of sequentially linked SEQ ID NO: 8, 32, 14, 18, and the FcB region;
or, amino acid sequence of the chain B consists of sequentially linked SEQ ID NO: 8, 32, 14, 19, and the FcB region;
or, amino acid sequence of the chain B consists of sequentially linked SEQ ID NO: 8, 32, 14, 20, and the FcB region;
or, amino acid sequence of the chain B consists of sequentially linked SEQ ID NO: 8, 32, 14, 21, and the FcB region;
or, amino acid sequence of the chain B consists of sequentially linked SEQ ID NO: 8, 32, 14, 22, and the FcB region;
or, amino acid sequence of the chain B consists of sequentially linked SEQ ID NO: 8, 32, 15, 17, and the FcB region;
or, amino acid sequence of the chain B consists of sequentially linked SEQ ID NO: 8, 32, 15, 18, and the FcB region;
or, amino acid sequence of the chain B consists of sequentially linked SEQ ID NO: 8, 32, 15, 19, and the FcB region;
or, amino acid sequence of the chain B consists of sequentially linked SEQ ID NO: 8, 32, 15, 20, and the FcB region;
or, amino acid sequence of the chain B consists of sequentially linked SEQ ID NO: 8, 32, 15, 21, and the FcB region;
or, amino acid sequence of the chain B consists of sequentially linked SEQ ID NO: 8, 32, 15, 22, and the FcB region;
or, amino acid sequence of the chain B consists of sequentially linked SEQ ID NO: 8, 32, 16, 17, and the FcB region;
or, amino acid sequence of the chain B consists of sequentially linked SEQ ID NO: 8, 32, 16, 18, and the FcB region;
or, amino acid sequence of the chain B consists of sequentially linked SEQ ID NO: 8, 32, 16, 19, and the FcB region;
or, amino acid sequence of the chain B consists of sequentially linked SEQ ID NO: 8, 32, 16, 20, and the FcB region;
or, amino acid sequence of the chain B consists of sequentially linked SEQ ID NO: 8, 32, 16, 21, and the FcB region;
or, amino acid sequence of the chain B consists of sequentially linked SEQ ID NO: 8, 32, 16, 22, and the FcB region.

In other embodiments of the present disclosure, when amino acid sequence of the chain A consists of sequentially linked SEQ ID NO: 9, 31, and the FcA region,
amino acid sequence of the chain B consists of sequentially linked SEQ ID NO: 10, 32, 13, 17, and the FcB region;
or, amino acid sequence of the chain B consists of sequentially linked SEQ ID NO: 10, 32, 13, 18, and the FcB region;
or, amino acid sequence of the chain B consists of sequentially linked SEQ ID NO: 10, 32, 13, 19, and the FcB region;
or, amino acid sequence of the chain B consists of sequentially linked SEQ ID NO: 10, 32, 13, 20, and the FcB region;
or, amino acid sequence of the chain B consists of sequentially linked SEQ ID NO: 10, 32, 13, 21, and the FcB region;
or, amino acid sequence of the chain B consists of sequentially linked SEQ ID NO: 10, 32, 13, 22, and the FcB region;
or, amino acid sequence of the chain B consists of sequentially linked SEQ ID NO: 10, 32, 14, 17, and the FcB region;
or, amino acid sequence of the chain B consists of sequentially linked SEQ ID NO: 10, 32, 14, 18, and the FcB region;
or, amino acid sequence of the chain B consists of sequentially linked SEQ ID NO: 10, 32, 14, 19, and the FcB region;
or, amino acid sequence of the chain B consists of sequentially linked SEQ ID NO: 10, 32, 14, 20, and the FcB region;
or, amino acid sequence of the chain B consists of sequentially linked SEQ ID NO: 10, 32, 14, 21, and the FcB region;
or, amino acid sequence of the chain B consists of sequentially linked SEQ ID NO: 10, 32, 14, 22, and the FcB region;
or, amino acid sequence of the chain B consists of sequentially linked SEQ ID NO: 10, 32, 15, 17, and the FcB region;
or, amino acid sequence of the chain B consists of sequentially linked SEQ ID NO: 10, 32, 15, 18, and the FcB region;
or, amino acid sequence of the chain B consists of sequentially linked SEQ ID NO: 10, 32, 15, 19, and the FcB region;
or, amino acid sequence of the chain B consists of sequentially linked SEQ ID NO: 10, 32, 15, 20, and the FcB region;
or, amino acid sequence of the chain B consists of sequentially linked SEQ ID NO: 10, 32, 15, 21, and the FcB region;
or, amino acid sequence of the chain B consists of sequentially linked SEQ ID NO: 10, 32, 15, 22, and the FcB region;
or, amino acid sequence of the chain B consists of sequentially linked SEQ ID NO: 10, 32, 16, 17, and the FcB region;
or, amino acid sequence of the chain B consists of sequentially linked SEQ ID NO: 10, 32, 16, 18, and the FcB region;
or, amino acid sequence of the chain B consists of sequentially linked SEQ ID NO: 10, 32, 16, 19, and the FcB region;
or, amino acid sequence of the chain B consists of sequentially linked SEQ ID NO: 10, 32, 16, 20, and the FcB region;
or, amino acid sequence of the chain B consists of sequentially linked SEQ ID NO: 10, 32, 16, 21, and the FcB region;
or, amino acid sequence of the chain B consists of sequentially linked SEQ ID NO: 10, 32, 16, 22, and the FcB region.

In the above embodiment, when amino acid sequence of the FcA region is SEQ ID NO: 26, amino acid sequence of the FcB region is SEQ ID NO: 27. When amino acid sequence of the FcA region is SEQ ID NO: 27, amino acid sequence of the FcB region is SEQ ID NO: 26.

In some embodiments of the present disclosure, the first functional binding region targets Trop-2; in the chain A, amino acid sequence of VH comprises SEQ ID NO: 11, amino acid sequence of VL comprises SEQ ID NO: 12, amino acid sequence of CH1 comprises SEQ ID NO: 31, amino acid sequence of CL comprises SEQ ID NO: 32, and amino acid sequence of the FcA region comprises SEQ ID NO: 26 or SEQ ID NO: 27;
in the chain B, amino acid sequence of VH comprises SEQ ID NO: 11, amino acid sequence of VL comprises SEQ ID NO: 12, amino acid sequence of CH1 comprises SEQ ID NO: 31, amino acid sequence of CL comprises SEQ ID NO: 32, amino acid sequence of the second functional binding region comprises any one of SEQ ID NOs: 17 to 22, and amino acid sequence of the FcB region comprises SEQ ID NO: 27 or SEQ ID NO: 26.

In some specific embodiments of the present disclosure, among the amino acid sequences of the chain B, C-terminus of SEQ ID NO: 12 or SEQ ID NO: 11 is linked to N-terminus of SEQ ID NO: 32 directly, C-terminus of SEQ ID NO: 32 is linked to N-terminus of any one of SEQ ID NOs: 17 to 22 via a linker sequence, and the linker sequence is selected from any one of SEQ ID NOs: 13 to 16.

In some embodiments of the present disclosure, when amino acid sequence of the chain A consists of sequentially linked SEQ ID NO: 11, 31, and the FcA region,
amino acid sequence of the chain B consists of sequentially linked SEQ ID NO: 12, 32, 13, 17, and the FcB region;
or, amino acid sequence of the chain B consists of sequentially linked SEQ ID NO: 12, 32, 13, 18, and the FcB region;
or, amino acid sequence of the chain B consists of sequentially linked SEQ ID NO: 12, 32, 13, 19, and the FcB region;
or, amino acid sequence of the chain B consists of sequentially linked SEQ ID NO: 12, 32, 13, 20, and the FcB region;
or, amino acid sequence of the chain B consists of sequentially linked SEQ ID NO: 12, 32, 13, 21, and the FcB region;
or, amino acid sequence of the chain B consists of sequentially linked SEQ ID NO: 12, 32, 13, 22, and the FcB region;
or, amino acid sequence of the chain B consists of sequentially linked SEQ ID NO: 12, 32, 14, 17, and the FcB region;
or, amino acid sequence of the chain B consists of sequentially linked SEQ ID NO: 12, 32, 14, 18, and the FcB region;
or, amino acid sequence of the chain B consists of sequentially linked SEQ ID NO: 12, 32, 14, 19, and the FcB region;
or, amino acid sequence of the chain B consists of sequentially linked SEQ ID NO: 12, 32, 14, 20, and the FcB region;
or, amino acid sequence of the chain B consists of sequentially linked SEQ ID NO: 12, 32, 14, 21, and the FcB region;
or, amino acid sequence of the chain B consists of sequentially linked SEQ ID NO: 12, 32, 14, 22, and the FcB region;
or, amino acid sequence of the chain B consists of sequentially linked SEQ ID NO: 12, 32, 15, 17, and the FcB region;
or, amino acid sequence of the chain B consists of sequentially linked SEQ ID NO: 12, 32, 15, 18, and the FcB region;
or, amino acid sequence of the chain B consists of sequentially linked SEQ ID NO: 12, 32, 15, 19, and the FcB region;
or, amino acid sequence of the chain B consists of sequentially linked SEQ ID NO: 12, 32, 15, 20, and the FcB region;
or, amino acid sequence of the chain B consists of sequentially linked SEQ ID NO: 12, 32, 15, 21, and the FcB region;
or, amino acid sequence of the chain B consists of sequentially linked SEQ ID NO: 12, 32, 15, 22, and the FcB region;
or, amino acid sequence of the chain B consists of sequentially linked SEQ ID NO: 12, 32, 16, 17, and the FcB region;
or, amino acid sequence of the chain B consists of sequentially linked SEQ ID NO: 12, 32, 16, 18, and the FcB region;
or, amino acid sequence of the chain B consists of sequentially linked SEQ ID NO: 12, 32, 16, 19, and the FcB region;
or, amino acid sequence of the chain B consists of sequentially linked SEQ ID NO: 12, 32, 16, 20, and the FcB region;
or, amino acid sequence of the chain B consists of sequentially linked SEQ ID NO: 12, 32, 16, 21, and the FcB region;
or, amino acid sequence of the chain B consists of sequentially linked SEQ ID NO: 12, 32, 16, 22, and the FcB region.

In the above embodiment, when amino acid sequence of the FcA region is SEQ ID NO: 26, amino acid sequence of the FcB region is SEQ ID NO: 27. When amino acid sequence of the FcA region is SEQ ID NO: 27, amino acid sequence of the FcB region is SEQ ID NO: 26.

In some embodiments of the present disclosure, when the first functional binding region targets GPC3, and amino acid sequence of the chain 1 in the first arm consists of sequentially linked SEQ ID NO: 1, 31, and the FcA region, amino acid sequence of the chain 2 in the first arm consists of sequentially linked SEQ ID NO: 2 and 32, and amino acid sequence of the second arm consists of sequentially linked any one of SEQ ID NOs: 17 to 22 and the FcB region; when amino acid sequence of the FcA region is SEQ ID NO: 26, amino acid sequence of the FcB region is SEQ ID NO: 27; or, when amino acid sequence of the FcA region is SEQ ID NO: 27, amino acid sequence of the FcB region is SEQ ID NO: 26.

In other embodiments of the present disclosure, when the first functional binding region targets PD-L1, and amino acid sequence of the chain 1 in the first arm consists of sequentially linked SEQ ID NO: 5, 31, and the FcA region, amino acid sequence of the chain 2 in the first arm consists of sequentially linked SEQ ID NO: 6 and 32, and amino acid sequence of the second arm consists of sequentially linked any one of SEQ ID NOs: 17 to 22 and the FcB region; when amino acid sequence of the FcA region is SEQ ID NO: 26, amino acid sequence of the FcB region is SEQ ID NO: 27; or, when amino acid sequence of the FcA region is SEQ ID NO: 27, amino acid sequence of the FcB region is SEQ ID NO: 26; or, when amino acid sequence of the FcA region is SEQ ID NO: 29, amino acid sequence of the FcB region is SEQ ID NO: 30; or, when amino acid sequence of the FcA region is SEQ ID NO: 30, amino acid sequence of the FcB region is SEQ ID NO: 29.

In other embodiments of the present disclosure, when the first functional binding region targets BCMA, and amino acid sequence of the chain 1 in the first arm consists of sequentially linked SEQ ID NO: 7, 31, and the FcA region, amino acid sequence of the chain 2 in the first arm consists of sequentially linked SEQ ID NO: 8 and 32; or, when amino acid sequence of the chain 1 in the first arm consists of sequentially linked SEQ ID NO: 9, 31, and the FcA region, amino acid sequence of the chain 2 in the first arm consists of sequentially linked SEQ ID NO: 10, 32; amino acid sequence of the second arm consists of sequentially linked any one of SEQ ID NOs: 17 to 22 and the FcB region; when amino acid sequence of the FcA region is SEQ ID NO: 26, amino acid sequence of the FcB region is SEQ ID NO: 27; or, when amino acid sequence of the FcA region is SEQ ID NO: 27, amino acid sequence of the FcB region is SEQ ID NO: 26.

In other embodiments of the present disclosure, when the first functional binding region targets Trop-2, and amino acid sequence of the chain 1 in the first arm consists of sequentially linked SEQ ID NO: 11, 31, and the FcA region, amino acid sequence of the chain 2 in the first arm consists of sequentially linked SEQ ID NO: 12 and 32, and amino acid sequence of the second arm consists of sequentially linked any one of SEQ ID NOs: 17 to 22 and the FcB region; when amino acid sequence of the FcA region is SEQ ID NO: 26, amino acid sequence of the FcB region is SEQ ID NO: 27; or, when amino acid sequence of the FcA region is SEQ ID NO: 27, amino acid sequence of the FcB region is SEQ ID NO: 26.

The third aspect of the present disclosure provides a nucleic acid molecule encoding the bispecific recombinant protein as described in the first or second aspect.

The fourth aspect of the present disclosure provides a recombinant expression vector comprising the nucleic acid molecule as described in the third aspect.

The fifth aspect of the present disclosure provides a recombinant cell obtained by transforming a host cell with the expression vector as described in the fourth aspect.

The sixth aspect of the present disclosure provides a method for preparing the bispecific recombinant protein as described in the first or second aspect, including the step of obtaining the bispecific recombinant protein by expression using the recombinant cell as described in the fifth aspect.

The seventh aspect of the present disclosure provides a medicament or pharmaceutical composition comprising the bispecific recombinant protein as described in the first or second aspect.

In some embodiments of the present disclosure, the pharmaceutical composition further comprises a pharmaceutically acceptable carrier.

The eighth aspect of the present disclosure provides a use of the bispecific recombinant protein as described in the first or second aspect or the medicament or pharmaceutical composition as described in the seventh aspect in the preparation of a medicament for the treatment, adjuvant therapy, or prevention of a disease.

In some embodiments of the disclosure, the disease is selected from the group consisting of tumors, liver diseases, and viral infectious diseases.

In some specific embodiments of the present disclosure, the disease is a GPC3, BCMA, PD-L1, Trop-2, 5T4, AGS-16, ALK1, ANG-2, B7-H3, B7-H4, c-fms, c-Met, CA6, CD123, CD19, CD20, CD22, CD24, EpCAM, CD30, CD32b, CD37, CD38, CD40, CD52, CD70, CD74, CD79b, CD98, CEA, CEACAM5, CLDN18.2, CLDN6, CS1, CXCR4, DLL-4, EGFR, EGFRvIII, EGP-1, ENPP3, EphA3, ETBR, FGFR2, FN, FR-α, GCC, GD2, GPNMB, HER2, HER3, HLA-DR, ICAM-1, IGF-1R, IL-3R, LIV-1, MSLN, MUC16, MUC1, NaPi2b, Nectin-4, Notch 2, Notch 1, PD-L2, PDGFR-α, PS, PSMA, SLTRK6, STEAP1, TEM1, VEGFR, CD25, CD27L, DKK-1, CSF-1R, MSB0010718C, CD138, Siglec15, or CD155-positive related disease.

In some embodiments of the present disclosure, the tumor is selected from the group consisting of breast cancer, intestinal cancer, pancreatic cancer, esophageal cancer, ovarian cancer, stomach cancer, prostate cancer, renal cancer, cervical cancer, myeloma, lymphoma, leukemia, thyroid cancer, uterine cancer, bladder cancer, neuroendocrine tumor, head and neck cancer, liver cancer, nasopharyngeal cancer, testicular cancer, lung cancer, melanoma, skin cancer, sarcoma, glioma, mesothelioma, and myelodysplastic syndrome.

In some specific embodiments of the present disclosure, the intestinal cancer includes colorectal cancer, the ovarian cancer includes yolk sac tumor, the neuroendocrine tumor includes Merkel cell carcinoma, the lung cancer includes small cell lung cancer and non-small cell lung cancer, the skin cancer includes basal cell skin cancer and squamous cell skin cancer, the sarcoma includes dermatofibrosarcoma protuberans, the glioma includes glioblastoma, and the uterine cancer includes endometrial cancer and uterine sarcoma.

As used herein, the term "recombinant protein" refers to an artificially designed/constructed protein, rather than a naturally occurring protein. The word "recombinant" in the "recombinant protein" of the present disclosure does not represent its production mode, but is only used to indicate that the "recombinant protein" does not naturally occur. The recombinant protein of the present disclosure may be an expressed protein and may be an assembled protein.

As used herein, the term "antibody" generally refers to a protein comprising one or more polypeptides essentially encoded by immunoglobulin genes or immunoglobulin gene fragments. Immunoglobulin genes can include κ, λ, α, γ, δ, ε, and µ constant region genes, as well as countless immunoglobulin variable region genes. As used herein, light chains may be classified as κ or λ. Heavy chains may be classified as γ, µ, α, δ, or ε, which in turn define the immunoglobulin classes: IgG, IgM, IgA, IgD, and IgE, respectively. Antibodies used in the present application may have a structural unit comprising a tetramer. Each tetramer may consist of two pairs of identical polypeptide chains, each pair having one "light chain" (approximately 25 kD) and one "heavy chain" (approximately 50 to 70 kD). N-terminus of each member can define a variable region of approximately 100 to 110 or more amino acids, which is primarily responsible for antigen recognition. As used herein, the terms "light chain variable region (VL)" and "heavy chain variable region (VH)" generally refer to these regions of the light chain and heavy chain, respectively. Antibodies may exist as intact immunoglobulins or as many fully characterized fragments produced by digestion with various peptidases or by de novo expression (for a more detailed description of other antibody fragments, see Fundamental Immunology, edited by W. E. Paul, Raven Press, N.Y. (1993)).

As used herein, the term "first functional antigen" or "target antigen" refers to an antigen that binds to a first functional binding region.

As used herein, the term "second functional antigen" refers to an antigen that binds to a second functional binding region.

As used herein, the term "Fc region" (fragment crystallizable, Fc) consists of IgG constant regions CH2 and CH3 domains and a hinge region.

As used herein, the term "antigen-binding fragment" or "fab fragment" or "fab" consists of domains of the light chain variable region (VL), the light chain constant region (CL), the heavy chain variable region (VH), and the heavy chain constant region 1 (CH1), and may bind to antigen.

As used herein, the term "knobs-into-holes technology" or "knobs-into-holes" is using genetic engineering techniques to introduce different mutations in two CH3 domains of the heavy chain, thereby promoting the heterodimerization of the heavy chain. A knob is made on one heavy chain and a hole is made on the other heavy chain, and then the two heavy chains preferentially couple together to form an asymmetric antibody (Ridgway JB, et al. "Knobs-into-holes" engineering of antibody CH3 domains for heavy chain heterodimerization. Protein Engineering, 1996, 9(7): 617-621). As known to those skilled in the art, a plurality of knobs and/or holes can be made on one heavy chain, and correspondingly, a plurality of holes and/or knobs can be made on the other heavy chain.

The term "antigen specificity" refers to the selective recognition of a specific antigen or epitope thereof by an antigen-binding molecule.

As used herein, the term "substitution" or "replacement" or "mutation" when applied to amino acid residues refers to the substitution of one or more naturally occurring or introduced amino acids with another in a peptide, polypeptide, or protein to form a new peptide, polypeptide, or protein (as termed herein "mutant"). Substitutions in a polypeptide or protein can result in weakened or unchanged function of the polypeptide or protein. Substitution may also be "conservative substitution", which in the context of an amino acid sequence refers to the substitution of one amino acid residue with another different amino acid residue in a side chain with similar physicochemical properties, or the substitution of amino acids that are not critical to the activity of the polypeptide. For example, conservative substitutions can occur between non-polar side chain amino acid residues (e.g., Met, Ala, Val, Leu, Ile, Pro, Phe, and Trp), between uncharged polar side chain residues (e.g., Cys, Ser, Thr, Asn, Gly, and Gin), between acidic side chain residues (e.g., Asp and Glu), between basic side chain amino acids (e.g., His, Lys, and Arg), between β-branched side chain amino acids (e.g., Thr, Val, and Ile), between sulfur-containing side chain amino acids (e.g., Cys and Met), or between aromatic side chain residues (e.g., Trp, Tyr, His, and Phe). In some embodiments, substitution, deletion or addition may also be considered as "conservative substitution". The number of inserted or deleted amino acids may range from approximately 1 to 5. Conservative substitutions generally do not cause significant changes in the conformational structure of the protein, and thus maintain the biological activity of the protein.

As used herein, the term "double-positive expressing cell" or "target cell" or "target target cell" refers to a cell that can interact with the first functional binding region and the second functional binding region simultaneously.

As used herein, the term "second functional antigen single-positive cell" or "non-target cell" or "non-target target cell" refers to cells that do not interact with the first functional binding region, but only with the second functional binding region.

As used herein, the term "interferon" (IFN) generally refers to a signaling protein that is produced and released by a host cell in response to the presence of a pathogen (e.g., a virus, a bacterium, a parasite, or a tumor cell). There are three main types of interferons (i.e., type I, type II, and type III), among which type I interferons may include IFNα and IPNβ, and IFNα may also include IFNα subtypes, for example IFNα2 and IFNα4. Type I interferons can inhibit viral replication, have anti-parasitic activity, inhibit cell proliferation, stimulate the cytotoxic activity of immune cells, participate in immune regulation, and exhibit anti-tumor effects. Type II and III interferons may include IFNγ, IPNλ1 (IL-29), IPNλ2 (IL-28a), and IPNλ3 (IL-28b). As used herein, the term "interferon" may include a full-length interferon or a fragment (e.g., a truncated form) or variant thereof, and the fragment or variant thereof can bind to a corresponding interferon receptor and retain its biological activity of cell proliferation inhibitory. As used herein, the interferon may be derived from any mammalian species. In some embodiments, the interferon is derived from a species selected from the group consisting of human, horse, cattle, mouse, pig, rabbit, cat, dog, rat, goat, sheep, and non-human primate.

As used herein, the term "IFNα" or "type α interferon" includes all natural or recombinant type α interferons, particularly preferably human type α interferons, for example, recombinant human type α interferons including, but not limited to IFN-α 2b (e.g., Intron^{®} A interferon or Viraferon^{®}-A interferon available from Schering Corporation, Kenilworth, N.J.), IFN-α 2a (e.g., Roferon^{®}-A interferon available from Hoffmann-La Roche, Nutley, N.J.); for another example, a mixture of natural type α interferons including, but not limited toIFN-α-n1 (e.g., Wellferon^{®} Interferon α-n1 available from Sumitomo, Japan or Glaxo-Wellcome Ltd., London, Great Britain) or IFN-α-n3 (e.g., Alferon N^{®} interferon available from Interferon Sciences). In the present disclosure, the term "IFNα" or "type α interferon" also include any substance having the biological activity of IFNα, for example, mutated or modified IFNα, for example a PEG derivative of IFNα (PEG-IFNα). In the present disclosure, the term "IFNα" or "type α interferon" is not limited to any specific source of acquisition, and can be obtained from commercially available sources or produced by conventional techniques known to those skilled in the art. The methods of production include, but not limited to, biological source extraction methods and genetic engineering extraction methods, which are described in detail in, for example, "Pestka S. Arch Biochem Biophys. 1983 Feb 15; 221(1): 1-37". In some embodiments, IFNα is derived from a species selected from the group consisting of human, horse, cattle, mouse, pig, rabbit, cat, dog, rat, goat, sheep, and non-human primate.

As used herein, the term "IFNα2b" or "IFN-α2b" or "IFN-α 2b" or "interferon-α 2b" is a subtype of IFN-α, all referring to interferon-α 2b. Amino acid sequence of human wild-type interferon-α 2b containing signal peptide is shown in SEQ ID NO: 15.

As used herein, the term "low-affinity mutant" refers to an IFNα mutant whose proliferation inhibitory or antiviral functional activity is unchanged or decreased compared to wild-type IFNα.

As used herein, the term "linker sequence" or "Linker" refers to an amino acid sequence that links different functional binding fragments (e.g., a first functional binding region and a second functional binding region, a first functional binding region or a second functional binding region and an Fc region), or links different domains within the same functional binding fragment.

As used herein, the terms "GC33", "Codrituzumab", and "Anti-GPC3 mAb" are used interchangeably in the present disclosure to refer to the anti-GPC3 antibody Codrituzumab.

As used herein, the terms "atezolizumab", "Atezolizumab", and "Anti-PD-L1 mAb" are used interchangeably in the present disclosure to refer to the anti-PD-L1 antibody Atezolizumab.

As used herein, the term "host cell" generally includes a single cell, cell line, or cell culture that can be or has been the recipient of a subject plasmid or vector, which contains a polynucleotide disclosed herein, or expresses a protein heterodimer (e.g., a heterodimeric protein) of the present disclosure. The host cell may comprise a progeny of a single host cell. The progeny may not necessarily be identical (either morphologically or in total genomic DNA complement) to the original parental cell due to natural, accidental, or intentional mutations. The host cell may include cells transfected *in vitro* with vectors disclosed herein. The host cell may be a bacterial cell (e.g., *E. coli*), yeast cell, or other eukaryotic cell, for example, HEK293 cell, COS cell, Chinese Hamster Ovary (CHO) cell, HeLa cell, or myeloma cell. In some embodiments, the host cell is a mammalian cell. In some embodiments, the mammalian cell is a CHO cell.

As used herein, the term "vector" generally refers to a nucleic acid molecule that is capable of self-replication in a suitable host and transfers an inserted nucleic acid molecule into host cells and/or between host cells. The term may include vectors primarily used for the insertion of DNA or RNA into cells, vectors primarily used for the replication of DNA or RNA, and expression vectors used for the transcription and/or translation of DNA or RNA. The term further includes vectors that provide more than one of the above functions. An "expression vector" is a polynucleotide that can be transcribed and translated into a polypeptide when introduced into a suitable host cell. An "expression system" generally means a suitable host cell containing an expression vector capable of producing the desired expression yield.

The term "effective amount" or "therapeutically effective amount" refers to an amount of a composition (e.g., a bispecific recombinant protein as described herein) that is sufficient to achieve the intended application (including, but not limited to, disease treatment). The therapeutically effective amount may vary depending on the intended application (e.g., *in vitro* or *in vivo*) or the subject and disease condition being treated, for example, the weight and age of the subject, the severity of the disease condition, the mode of administration, etc., which can be readily determined by those skilled in the art. The term may also be applied to a dose that induces a specific response (e.g., target gene induction, proliferation, and/or apoptosis) in a target cell. The specific dose will vary depending on the specific compound selected, the dosage regimen followed, whether it is administered in combination with other compounds, the timing of administration, the tissue to which it is administered, and the physical delivery system in which it is located.

The terms "treatment", "cure", "mitigation", and "improvement" are used interchangeably herein and refer to methods of obtaining beneficial or desired results (including, but not limited to, therapeutic benefits and/or preventive benefits). As used herein, the therapeutic benefit generally refers to eradication or alleviation of the severity of the underlying condition being treated. In addition, the therapeutic benefit is achieved by eradicating and alleviating the severity, or reducing the incidence of one or more physiological symptoms associated with the underlying condition in such a way as to allow improvement to be observed in the subject (even though the subject may still be afflicted with the underlying condition). For preventive benefits, the composition may be administered to a subject who is at risk of developing a particular disease, or who reports one or more physiological symptoms of a disease, even though a diagnosis of the disease may not have been made.

As used herein, the term "therapeutic effect" generally includes therapeutic benefits and/or preventive benefits as described above. The preventive effects include delaying or eliminating the occurrence of a disease or condition, delaying or eliminating the onset of symptoms of a disease or condition, slowing, halting, or reversing the progression of a disease or condition, or any combination thereof.

As used herein, the terms "co-administration", "administration in combination with", and their grammatical equivalents generally include the administration of two or more agents to an animal such that the agents and/or their metabolites are present in the subject simultaneously. Co-administration includes simultaneous administration in separate compositions, administration at different times in separate compositions, or administration in a composition in which both agents are present.

As used herein, the term "cell proliferation" generally refers to the phenomenon in which the number of cells changes due to division. For example, cell proliferation can lead to an increase in the number of cells. The term further includes cell growth by which the cell morphology has been altered (e.g., increased in size), which is consistent with proliferative signaling.

As used herein, the term "proliferation inhibition" or "inhibition of cell proliferation" generally refers to a reduction in the growth rate and/or proliferation rate of cancer cells. For example, this may include the death of cancer cells (e.g., by apoptosis). In some embodiments, the term may further refer to inhibiting the growth and/or proliferation of a solid tumor and/or inducing a reduction in size or elimination of a tumor.

As used herein, the term "freeze-thaw stability" generally refers to the stability of an emulsion system when subjected to alternating freezing and thawing.

The term "subject", "individual", "animal", or "patient" as used herein refers to a human or non-human animal, including a mammal or primate, who requires diagnosis, prognosis, mitigation, prevention, and/or treatment of a disease or condition. A mammalian subject includes a human, livestock animal, farm animal, and zoo, sport, or pet animal, for example, a dog, cat, guinea pig, rabbit, rat, mouse, horse, pig, cow, and bear.

As used herein, the term "*in vivo*" generally refers to an event that occurs within the body of a subject.

As used herein, the term "*in vitro*" generally refers to an event that occurs outside the body of a subject. For example, *in vitro* assays include any assay performed outside the body of a subject. *In vitro* assays include cell-based assays in which dead or live cells are used. *In vitro* assays also include cell-free assays in which intact cells are not used.

As used herein, the term "administration" refers to the delivery of a therapeutically effective amount of a pharmaceutical composition comprising a recombinant protein or fusion protein of the present disclosure to a subject. The administration can be systemic or topical. The administration can be performed via an administration device, for example, a syringe. Modes of administration include, but are not limited to, embedding, nasal inhalation, spraying, injecting, etc. Routes of administration include inhalation, intranasal, oral, intravenous, subcutaneous, or intramuscular administration, etc.

Compared to the prior art, the present disclosure has the following beneficial effects:

The present disclosure provides a new bispecific recombinant protein having two types of structures, comprising an interferon, a truncated variant thereof, or a mutant thereof, which has high activity along with high safety and freeze-thaw stability.

For cells expressing IFNα receptors, the beneficial effects are as follows:
1. The bispecific recombinant proteins of the present disclosure all have significantly stronger binding affinity, ADCC activity, and proliferation inhibitory activity on target antigen-positive target cells than that of IFNα at an equimolar concentration, and have non-significantly weaker binding affinity and proliferation inhibitory activity on target antigen-negative non-target cells (e.g., normal cells) than that of IFNα at an equimolar concentration, and some experiments even have negative results of the proliferation inhibitory activity of the bispecific recombinant protein on non-target cells. That is, when the bispecific recombinant protein of the present disclosure does not have the ability to target the target antigen, IFNα in the second functional binding region has significantly reduced activity or even no relevant activity on cells expressing the IFNα receptor.
2. The bispecific recombinant proteins of the present disclosure having the first type of structure, in which C-terminus of the CH1 domain or C-terminus of the CL domain is linked to N-terminus of the second functional binding region via different linker sequences in the antigen-binding fragment all have strong proliferation inhibitory activity on target antigen-positive target cells, and the proliferation inhibitory activity (IC₅₀) is higher than that of the interferon control; the bispecific recombinant protein with a shorter linker sequence has relatively weak activity, and the activity of the second functional binding region on cells expressing IFNα receptor can thereby be modulated by adjusting the length of the linker sequence.
3. The decrease in the proliferation inhibitory activity of the bispecific recombinant protein of the present disclosure containing IFN-α 2b low-affinity mutant on target antigen-negative non-target cells relative to the proliferation inhibitory activity of the bispecific recombinant protein containing wild-type IFN-α 2b on target antigen-negative non-target cells is comparable to or more significant than the decrease in the proliferation inhibitory activity of the bispecific recombinant protein containing IFN-α 2b low-affinity mutant on target antigen-positive target cells.
4. The potentially risky impurities (chain B or second arm homodimers) of the bispecific recombinant protein of the present disclosure have extremely weak proliferation inhibitory activity on target antigen-negative non-target cells, wherein the potentially risky impurities of the bispecific recombinant protein having the first type of structure have weaker proliferation inhibitory activity on target antigen-negative non-target cells compared to the potentially risky impurities of the bispecific recombinant protein having the second type of structure. Therefore, the potentially risky impurities of the bispecific recombinant protein of the present disclosure have extremely low potential safety risks or potential toxic side effects. The potentially risky impurities of the bispecific recombinant protein having the first type of structure have lower potential safety risks or potential toxic side effects.
5. The bispecific recombinant protein of the present disclosure has good freeze-thaw stability. After 5 repeated freeze-thaw cycles, it has a purity of above 95%, a clear appearance, and significantly better freeze-thaw stability than that of IFN-α 2b monomer or PEGylated IFN-α 2b.
6. The bispecific recombinant protein of the present disclosure has strong extensibility in structure, and its first functional binding region can be used to construct antigen-binding fragments of specific antibodies targeting the target antigen according to the needs of clinical indications, which significantly reduces the time spent on conventional antibody drug screening, improves the efficiency of drug screening, and reduces the cost of screening.

In summary, the bispecific recombinant protein of the present disclosure can significantly improve the killing effect on of target target cells, while significantly reduce the toxic side effects caused by binding to non-target target cells, and has better safety and good freeze-thaw stability. The conception, specific structure, and resulting technical effects of the present disclosure will be further described hereinafter in conjunction with the accompanying drawings, so as to fully understand the purpose, features, and effects of the present disclosure.

### BRIEF DESCRIPTION OF THE DRAWINGS

Figure 1 shows a structural schematic diagram of the bispecific recombinant protein having the first type of structure of the present disclosure.
Figure 2 shows a structural schematic diagram of the bispecific recombinant protein having the second type of structure of the present disclosure.
Figure 3 shows a non-reducing SDS-PAGE electropherogram of the bispecific recombinant protein having the first type of structure of the present disclosure after affinity capture.
Figure 4 shows a reducing SDS-PAGE electropherogram of the bispecific recombinant protein having the first type of structure of the present disclosure after affinity capture.
Figure 5 shows a non-reducing SDS-PAGE electropherogram of the bispecific recombinant protein having the first type of structure of the present disclosure after secondary purification.
Figure 6 shows a non-reducing SDS-PAGE electropherogram of the bispecific recombinant protein having the second type of structure of the present disclosure after Kappa purification.
Figure 7 shows a graph of the binding affinity of the bispecific recombinant protein (the first type of structure) of the present disclosure and the control sample to target cells (HepG2 liver cancer cells) determined by flow cytometry.
Figure 8 shows a histogram of the binding affinity of the bispecific recombinant protein (the first type of structure) of the present disclosure and the control sample to target cells HuH-7 determined by flow cytometry.
Figure 9 shows a graph of the binding affinity of the bispecific recombinant protein (the second type of structure) of the present disclosure and the control sample to target cells HepG2 determined by flow cytometry.
Figure 10 shows a graph of the ADCC activity of the bispecific recombinant protein (the first type of structure) of the present disclosure to target cells HepG2 determined by the LDH method.
Figure 11 shows a graph of the ADCC activity of the bispecific recombinant protein (the second type of structure) of the present disclosure and the control sample to target cells HepG2determined by the LDH method.
Figure 12 shows a graph of the proliferation inhibitory activity of the bispecific recombinant protein (the first type of structure) with different linker sequences of the present disclosure on target cells HuH-7.
Figure 13 shows a graph of the proliferation inhibitory activity of the bispecific recombinant protein (the first type of structure) with the function of targeting GPC3 antigen and the control sample on target cells HuH-7.
Figure 14 shows a graph of the proliferation inhibitory activity of the bispecific recombinant protein (the first type of structure) of the present disclosure on PD-L1-positive target cells MDA-MB-231.
Figure 15 shows a graph of the proliferation inhibitory activity of the bispecific recombinant protein (the first type of structure) of the present disclosure on MDA-MB-231 blocked by anti-PD-L1 antibody.
Figure 16 shows a graph of the proliferation inhibitory activity of the bispecific recombinant protein (the first type of structure) containing different IFN-α2b low-affinity mutants of the present disclosure on GPC3-positive target cells HuH-7.
Figure 17 shows a graph of the proliferation inhibitory activity of the bispecific recombinant protein (the first type of structure) containing different IFN-α2b low-affinity mutants of the present disclosure on GPC3-negative non-target cells SW480.
Figure 18 shows a graph of the proliferation inhibitory activity of the bispecific recombinant protein (the first type of structure) containing different IFN-α2b low-affinity mutants of the present disclosure on GPC3-negative non-target cells U266.
Figure 19 shows a graph of the proliferation inhibitory activity of the bispecific recombinant protein (the first type of structure) containing different IFN-α2b low-affinity mutants of the present disclosure on Trop-2-positive target cells OVCAR3.
Figure 20 shows a graph of the proliferation inhibitory activity of the bispecific recombinant protein (the first type of structure) containing different IFN-α2b low-affinity mutants of the present disclosure on Trop-2-positive target cells MDA-MB-231.
Figure 21 shows a graph of the proliferation inhibitory activity of the bispecific recombinant protein (the first type of structure) containing different IFN-α2b low-affinity mutants of the present disclosure on Trop-2-negative non-target cells NCI-H929.
Figure 22 shows a graph of the proliferation inhibitory activity of the bispecific recombinant protein (the first type of structure) containing different IFN-α2b low-affinity mutants of the present disclosure on BCMA-positive target cells NCI-H929.
Figure 23 shows a graph of the proliferation inhibitory activity of the bispecific recombinant protein (the first type of structure) containing different IFN-α2b low-affinity mutants of the present disclosure on BCMA-negative non-target cells HuH-7.
Figure 24 shows a graph of the proliferation inhibitory activity of the bispecific recombinant protein (the first type of structure) containing different IFN-α2b low-affinity mutants of the present disclosure on BCMA-negative non-target cells MDA-MB-231.
Figure 25 shows a graph of the proliferation inhibitory activity of the bispecific recombinant protein (the first type of structure) containing different IFN-α2b low-affinity mutants of the present disclosure on PD-L1-positive target cells MDA-MB-231.
Figure 26 shows a graph of the proliferation inhibitory activity of the bispecific recombinant protein (the first type of structure) containing different IFN-α2b low-affinity mutants of the present disclosure on PD-L1-negative target cells OVCAR-3.
Figure 27 shows a graph of the proliferation inhibitory activity of the bispecific recombinant protein (the second type of structure) containing different IFN-α2b low-affinity mutants of the present disclosure on GPC3-positive target cells HuH-7.
Figure 28 shows a graph of the proliferation inhibitory activity of the bispecific recombinant protein (the second type of structure) containing different IFN-α2b low-affinity mutants of the present disclosure on GPC3-negative non-target cells SW480.
Figure 29 shows a graph of the proliferation inhibitory activity of the bispecific recombinant protein (the second type of structure) containing different IFN-α2b low-affinity mutants of the present disclosure on GPC3-negative non-target cells MDA-MB-231.
Figure 30 shows a graph of the proliferation inhibitory activity of the bispecific recombinant protein (the second type of structure) containing different IFN-α2b low-affinity mutants of the present disclosure on GPC3-negative non-target cells U266.
Figure 31 shows a graph of the proliferation inhibitory activity of the potentially risky impurities of the bispecific recombinant protein of the present disclosure on GPC3-negative non-target cells MDA-MB-231.
Figure 32 shows a graph of the anti-tumor efficacy of the bispecific recombinant protein of the present disclosure on the HuH-7 subcutaneous tumor model in BALB/c nude mice.
Figure 33 shows a histogram of the anti-tumor efficacy of the bispecific recombinant protein of the present disclosure on the HuH-7 subcutaneous tumor model in BALB/c nude mice.
Figure 34 shows a histogram of the anti-tumor efficacy of the bispecific recombinant protein of the present disclosure on the MDA-MB-231 model in PBMC humanized M-NSG mice.

### DETAILED DESCRIPTION OF THE PREFERRED EMBODIMENT

In order to make it easy to understand the technical means, creative features, objectives, and effects of the present disclosure, the present disclosure is further elaborated below with reference to specific illustrations. However, the present disclosure is not limited to the following examples.

### Example 1. Design of bispecific recombinant protein

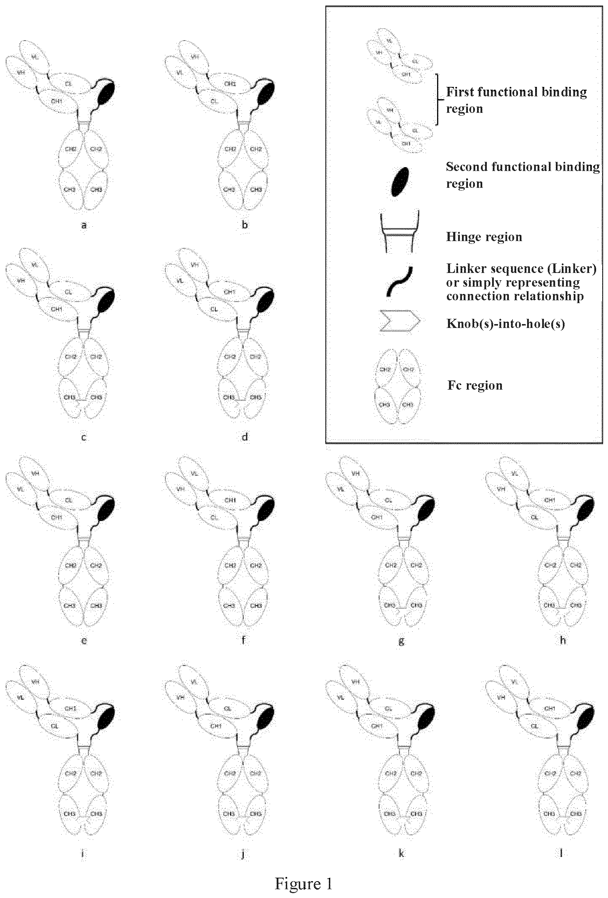

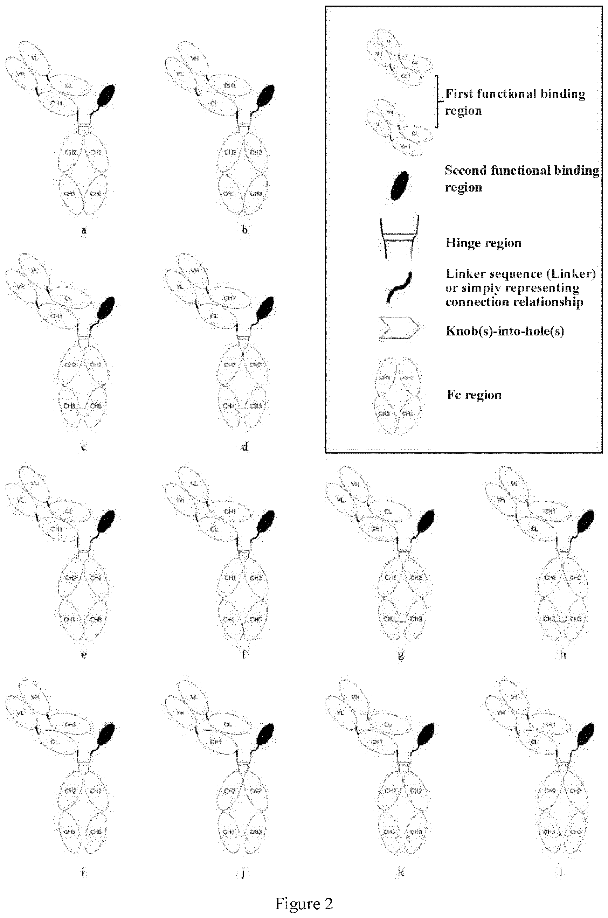

The structure of the bispecific recombinant protein of the present disclosure is shown in Figure 1 and Figure 2. The bispecific recombinant protein comprises a first functional binding region targeting the target antigen, a second functional binding region consisting of IFNα a low-affinity mutant thereof, a truncated variant retaining the IFNα function, or a mutant thereof, and an Fc region. Among them, C-terminus of the second functional binding region is linked to the Fc region directly or via a linker sequence, and C-terminus of the first functional binding region is linked to the Fc region directly or via a linker sequence; variable region (V region) and constant region (C region) of the first functional binding region are linked directly or via a linker sequence; the Fc region can be a conventional IgG structure, and knobs-into-holes technology can be used; the first functional binding region and the second functional binding region are linked in two structures, namely:
a first type of structure, in which N-terminus of the second functional binding region and C-terminus of CL or CH1 in the first functional binding region that is not linked to the Fc region are linked directly or via a linker sequence (as shown in Figure 1);
a second type of structure, in which N-terminus of the second functional binding region is separated from the first functional binding region, that is, N-terminus of the second functional binding region is not linked to any other functional fragments (except signal peptide) (as shown in Figure 2).

In this example, the second functional binding region selected from human IFN-α 2b (Genebank: AAP20099.1) is used as an example to illustrate the design of the bispecific recombinant protein of the present disclosure. The IFNα family consists of 15 subtypes. Different subtypes have similar structures, high sequence homology (80 to 99%), and bind to the same IFN receptor, all of which have antiviral, proliferation inhibitory, anti-tumor, and immunomodulatory functions. The technical effects obtainable with IFN-α 2b will not be repeated herein, as consistent technical effects can be achieved with the other IFNα subtypes (British Journal of Pharmacology (2013) 168 1048-1058).

The bispecific recombinant proteins having the first type of structure are shown in Table 1, the bispecific recombinant proteins having the second type of structure are shown in Table 2, and the control samples are shown in Table 3.

**Table 1: Exemplary molecular structures of bispecific recombinant proteins having the first type of structure**

| Bispecific recombinant protein No. | First functional antibody × second functional protein | Exemplary bispecific antibody molecular composition | |
|---|---|---|---|
| | | Chain A | Chain B |
| TTM101-LC01 | Anti-GPC3 mAb × IFN-α 2b | GC33(H)-Fc1 | GC33(L)-(GGGGS)₁-IFNα2b-Fc2 |
| TTM101-LC02 | Anti-GPC3 mAb × IFN-α 2b | GC33(H)-Fc1 | GC33(L)-(GGGGS)₂-IFNα2b-Fc2 |
| TTM101-LC03 | Anti-GPC3 mAb × IFN-α 2b | GC33(H)-Fc1 | GC33(L)-(GGGGS)₄-IFNα2b-Fc2 |
| TTM101-LC02-M1 | Anti-GPC3 mAb × IFN-α 2b (L26A) | GC33(H)-Fc1 | GC33(L)-(GGGGS)₂-IFNa2b (L26A)-Fc2 |
| TTM101-LC02-M2 | Anti-GPC3 mAb × IFN-α 2b (L30A) | GC33(H)-Fc1 | GC33(L)-(GGGGS)₂-IFNa2b (L30A)-Fc2 |
| TTM101-LC02-M3 | Anti-GPC3 mAb × IFN-α 2b (A145G) | GC33(H)-Fc1 | GC33(L)-(GGGGS)₂-IFNa2b (A145G)-Fc2 |
| TTM101-LC02-M4 | Anti-GPC3 mAb × IFN-α 2b (R149A) | GC33(H)-Fc1 | GC33(L)-(GGGGS)₂-IFNa2b (R149A)-Fc2 |
| TTM101-LC02-M5 | Anti-GPC3 mAb × IFN-α 2b (S152A) | GC33(H)-Fc1 | GC33(L)-(GGGGS)₂-IFNa2b (S152A)-Fc2 |
| TTM101-LC03-M3 | Anti-GPC3 mAb × IFN-α 2b (A145G) | GC33(H)-Fc1 | GC33(L)-(GGGGS)₄-IFNa2b (A145G)-Fc2 |
| TTM101-LC03-M4 | Anti-GPC3 mAb × IFN-α 2b (R149A) | GC33(H)-Fc1 | GC33(L)-(GGGGS)₄-IFNa2b (R149A)-Fc2 |
| TTM101-LC04 | Anti-RSV mAb × IFN-α 2b | Palivizwnab(H)-Fcl | Palivizumab(L)-(GGGGS)₂-IFNα2b-Fc2 |
| TTM101-LC05 | Anti-PD-L1 mAb × IFN-α 2b | Atezolizumab(H)-Fc1 | Atezofizumab(L)-(GGGGS)₁-IFNα2b-Fc2 |
| TTM101-LC06 | Anti-PD-L1 mAb × IFN-α 2b | Atezolizumab(H)-Fc1 | Atezofizumab(L)-(GGGGS)₂-IFNα2b-Fc2 |
| TTM101-LC07 | Anti-PD-L1 mAb × IFN-α 2b | Atezolizumab(H)-Fc1 | Atezofizumab(L)-(GGGGS)₃-IFNα2b-Fc2 |
| TTM101-LC05 (N297G) | Anti-PD-L1 mAb × IFN-α 2b | Atezolizumab(H)-Fc1 (N297G) | Atezofizumab(L)-(GGGGS)₁-IFNα2b-Fc2 (N297G) |
| TTM101-LC06 (N297G) | Anti-PD-L1 mAb × IFN-α 2b | Atezolizumab(H)-Fc1 (N297G) | Atezofizumab(L)-(GGGGS)₂-IFNα2b-Fc2 (N297G) |
| TTM101-LC07 (N297G) | Anti-PD-L1 mAb × IFN-α 2b | Atezolizumab(H)-Fc1 (N297G) | Atezofizumab(L)-(GGGGS)₃-IFNα2b-Fc2 (N297G) |
| TTM101-LC08-M3 | Anti-BCMA mAb × IFN-α 2b (A145G) | Elranatamab(H)-Fc 1 | Elranatamab(L)-(GGGGS)₄-IFNα2b (A145G)-Fc2 |
| TTM101-LC08-M4 | Anti-BCMA mAb × IFN-α 2b (R149A) | Elranatamab(H)-Fc 1 | Elranatamab(L)-(GGGGS)₄-IFNα2b (R149A)-Fc2 |
| TTM101-LC09-M3 | Anti-BCMA mAb × IFN-α 2b (A145G) | Belantamab(H)-Fc1 | Belantamab(L)-(GGGGS)₄-IFNα2b (A145G)-Fc2 |
| TTM101-LC09-M4 | Anti-BCMA mAb × IFN-α 2b (R149A) | Belantamab(H)-Fc1 | Belantamab(L)-(GGGGS)₄-IFNα2b (R149A)-Fc2 |
| TTM101-LC10 | Anti-Trop-2 mAb × IFN-α 2b | hRS7(H)-Fc1 | hRS7(L)-(GGGGS)₄-IFNα2b-Fc2 |
| TTM101-LC10-M3 | Anti-Trop-2 mAb × IFN-α 2b (A145G) | hRS7(H)-Fc1 | hRS7(L)-(GGGGS)₄-IFNa2b (A145G)-Fc2 |
| TTM101-LC10-M4 | Anti-Trop-2 mAb × IFN-α 2b (R149A) | hRS7(H)-Fc1 | hRS7(L)-(GGGGS)₄-IFNa2b (R149A)-Fc2 |
| TTM101-LC07-M3 | Anti-PD-L1 mAb × IFN-α 2b (A145G) | Atezolizumab(H)-Fc1 | Atezofizumab(L)-(GGGGS)₃-IFNα2b (A145G)-Fc2 |
| TTM101-LC07-M4 | Anti-PD-L1 mAb × IFN-α 2b (R149A) | Atezolizumab(H)-Fc1 | Atezofizumab(L)-(GGGGS)₃-IFNα2b (R149A)-Fc2 |
| TTM101-LC07-M3 (N297G) | Anti-PD-L1 mAb × IFN-α 2b (A145G) | Atezolizumab(H)-Fc1 (N297G) | Atezolizumab(L)-(GGGGS)₃-IFNα2b (A145G)-Fc2 (N297G) |
| TTM101-LC07-M4 (N297G) | Anti-PD-L1 mAb × IFN-α 2b (R149A) | Atezolizumab(H)-Fc1 (N297G) | Atezolizumab(L)-(GGGGS)₃-IFNα2b (R149A)-Fc2 (N297G) |

**Table 2: Exemplary molecular structures of bispecific recombinant proteins having the second type of structure**

| Bispecific recombinant protein No. | First functional antibody × second functional protein | Exemplary bispecific antibody molecular composition | |
|---|---|---|---|
| | | First arm | Second arm |
| TTM101-01 | Anti-GPC3 mAb × IFN-α 2b | GC33(H)-Fc1 + GC33(L) | IFNα2b-Fc2 |
| TTM101-01-M3 | Anti-GPC3 mAb × IFN-α 2b (A145G) | GC33(H)-Fc1 + GC33(L) | IFNa2b (A145G)-Fc2 |
| TTM101-01-M4 | Anti-GPC3 mAb × IFN-α 2b (R149A) | GC33(H)-Fc1 + GC33(L) | IFNa2b (R149A)-Fc2 |
| TTM101-03 | Anti-RSV mAb × IFN-α 2b | Palivizumab(H)-Fcl + Palivizumab(L) | IFNα2b-Fc2 |

**Table 3: Exemplary molecular structures of control samples**

| Control sample name | Antigen | Molecular composition |
|---|---|---|
| Codrituzumab | GPC3 | Anti-GPC3 monoclonal antibody Codrituzumab |
| Palivizumab | Respiratory syncytial virus (RSV) | Anti-RSV monoclonal antibody Palivizumab |
| IFN-α 2b-Fc | None | Recombinant expression IFN-α 2b and human IgG1 Fc fusion protein |
| IFN-α 2b | None | Recombinant expression IFN-α 2b protein |
| Human IgG1 isotype control (Isotype) | Hen egg lysozyme (HEL) | Anti-HEL monoclonal antibody as an isotype control |
| Atezolizumab | PD-L1 | Anti-PD-L1 monoclonal antibody Atezolizumab |
| Elranatamab | BCMA | Anti-BCMA monoclonal antibody Elranatamab |
| Belantamab | BCMA | Anti-BCMA monoclonal antibody Belantamab |
| hRS7 | Trop-2 | Anti-Trop-2 monoclonal antibody hRS7 |

In the above Table 1 and Table 2, (H) refers to a domain consisting of heavy chain VH and CH1, (L) refers to a domain consisting of light chain VL and CL; GC33 represents the anti-phosphatidylinositol proteoglycan (Glypican3, GPC3) monoclonal antibody Codrituzumab; Fc represents the wild-type Fc region, Fc1 represents the Fc region with hole or holes mutations, and Fc2 represents the Fc region with knob or knobs mutations.

The sequence numbers corresponding to the sequence names described in Table 1 and Table 2 are shown in Table 4-1 and Table 4-2. Among them, the chain A and chain B signal peptides having the first type of structure and the first arm (H+L) signal peptide having the second type of structure are shown in SEQ ID NO: 23; the second arm signal peptide having the second type of structure is shown in SEQ ID NO: 24, which is a natural signal peptide of IFNα2b. In Table 3, amino acid sequence of IFN-α 2b-Fc consists of sequentially linked SEQ ID NO: 17 and SEQ ID NO: 25, and sequence of the corresponding signal peptide is shown in SEQ ID NO: 24; amino acid sequence of IFN-α 2b is shown in SEQ ID NO: 17; amino acid sequence of Codrituzumab is cited from patent US7919086, wherein amino acid sequence of the heavy chain variable region is shown in SEQ ID NO: 1, and amino acid sequence of the light chain variable region is shown in SEQ ID NO: 2. Amino acid sequence of Atezolizumab is cited from patent US20100203056, wherein amino acid sequence of the heavy chain variable region is shown in SEQ ID NO: 5, and amino acid sequence of the light chain variable region is shown in SEQ ID NO: 6. Amino acid sequence of Palivizumab is cited from patent WO1994017105, wherein amino acid sequence of the heavy chain variable region is shown in SEQ ID NO: 3, and amino acid sequence of the light chain variable region is shown in SEQ ID NO: 4. Sequences of the heavy and light chain variable region of Belantamab are cited from patent US20190194338, wherein sequence of the heavy chain variable region is shown in SEQ ID NO: 9, and sequence of the light chain variable region is shown in SEQ ID NO: 10. Sequences of the heavy and light chain variable region of Palivizumab are cited from patent US20160297885, wherein sequence of the heavy chain variable region is shown in SEQ ID NO: 7, and sequence of the light chain variable region is shown in SEQ ID NO: 8. Sequences of the heavy and light chain variable region of hRS7 are cited from patent US7517964, wherein sequence of the heavy chain variable region is shown in SEQ ID NO: 11, and sequence of the light chain variable region is shown in SEQ ID NO: 12. Human IgG1 isotype control (B 117901) was purchased from Biointron. Recombinant expression of IFN-α 2b protein (Z03003) was purchased from Nanjing GenScript Biotech Co., Ltd.

**Table 4-1: Composition of partial sequence names and corresponding sequence numbers**

| Sequence name | SEQ ID NO |
|---|---|
| GC33(H)-Fc1 | 1+31+26 |
| GC33(L)-(GGGGS)₁-IFNα2b-Fc2 | 2+32+13+17+27 |
| GC33(L)-(GGGGS)₂-IFNα2b-Fc2 | 2+32+14+17+27 |
| GC33(L)-(GGGGS)₄-IFNα2b-Fc2 | 2+32+16+17+27 |
| GC33(L)-(GGGGS)₂-IFNα2b (L26A)-Fc2 | 2+32+14+18+27 |
| GC33(L)-(GGGGS)₂-IFNα2b (L30A)-Fc2 | 2+32+14+19+27 |
| GC33(L)-(GGGGS)₂-IFNα2b (A145G)-Fc2 | 2+32+14+20+27 |
| GC33(L)-(GGGGS)₂-IFNα2b (R149A)-Fc2 | 2+32+14+21+27 |
| GC33(L)-(GGGGS)₂-IFNα2b (S152A)-Fc2 | 2+32+14+22+27 |
| GC33(L)-(GGGGS)₄-IFNα2b (A145G)-Fc2 | 2+32+16+20+27 |
| GC33(L)-(GGGGS)₄-IFNα2b (R149A)-Fc2 | 2+32+16+21+27 |
| Palivizwnab(H)-Fc1 | 3+31+26 |
| Palivizumab(L)-(GGGGS)₂-IFNα2b-Fc2 | 4+32+14+17+27 |
| IFN-α 2b-Fc | 17+25 |
| IFN-α 2b | 17 |
| GC33(L) | 2+32 |
| Atezolizumab(H)-Fc (N297A) | 5+31+28 |
| Atezolizumab(L) | 6+32 |
| Palivizumab(H)-Fc | 3+31+25 |
| Palivizumab(L) | 4+32 |
| Atezolizumab(H)-Fc1 | 5+31+26 |
| Atezolizumab(L)-(GGGGS)₁-IFNα2b-Fc2 | 6+32+13+17+27 |
| Atezolizumab(L)-(GGGGS)₂-IFNα2b-Fc2 | 6+32+14+17+27 |
| Atezolizumab(L)-(GGGGS)₃-IFNα2b-Fc2 | 6+32+15+17+27 |
| Atezolizumab(H)-Fc1 (N297G) | 5+31+29 |
| Atezofizumab(L)-(GGGGS)₁-IFNα2b-Fc2 (N297G) | 6+32+13+17+30 |
| Atezolizumab(L)-(GGGGS)₂-IFNα2b-Fc2 (N297G) | 6+32+14+17+30 |
| Atezolizumab(L)-(GGGGS)₃-IFNα2b-Fc2 (N297G) | 6+32+15+17+30 |
| IFN-α 2b-Fc2 | 17+27 |
| IFN-α 2b (A145G)-Fc2 | 20+27 |
| IFN-α 2b (R149A)-Fc2 | 21+27 |
| Elranatamab(H)-Fc1 | 7+31+26 |
| Elranatamab(L)-(GGGGS)₄-IFNα2b (A145G)-Fc2 | 8+32+16+20+27 |
| Elranatamab(L)-(GGGGS)₄-IFNα2b (R149A)-Fc2 | 8+32+16+21+27 |
| Belantamab(H)-Fc1 | 9+31+26 |
| Belantamab(L)-(GGGGS)₄-IFNα2b (A145G)-Fc2 | 10+32+16+20+27 |
| Belantamab(L)-(GGGGS)₄-IFNα2b (R149A)-Fc2 | 10+32+16+21+27 |
| hRS7(H)-Fc1 | 11+31+26 |
| hRS7(L)-(GGGGS)₄-IFNα2b-Fc2 | 12+32+16+17+27 |
| hRS7(L)-(GGGGS)₄-IFNα2b (A145G)-Fc2 | 12+32+16+20+27 |
| hRS7(L)-(GGGGS)₄-IFNα2b (R149A)-Fc2 | 12+32+16+21+27 |
| Atezolizumab(L)-(GGGGS)₃-IFNα2b (A145G)-Fc2 | 6+32+15+20+27 |
| Atezolizumab(L)-(GGGGS)₃-IFNα2b (R149A)-Fc2 | 6+32+15+21+27 |
| Atezolizumab(L)-(GGGGS)₃-IFNα2b (A145G)-Fc2(N297G) | 6+32+15+20+30 |
| Atezolizumab(L)-(GGGGS)₃-IFNα2b (R149A)-Fc2(N297G) | 6+32+15+21+30 |

**Table 4-2: Composition of other sequence names and corresponding sequence numbers**

| Sequence name | SEQ ID NO | Sequence |
|---|---|---|
| GC33(VH) | 1 | QVQLVQSGAEVKKPGASVKVSCKASGYTFTDYEMHWVR QAPGQGLEWMGALDPKTGDTAYSQKFKGRVTLTADKSTS TAYMELSSLTSEDTAVYYCTRFYSYTYWGQGTLVTVSS |
| GC33(VL) | 2 | DVVMTQSPLSLPVTPGEPASISCRSSQSLVHSNRNTYLHWY LQKPGQSPQLLIYKVSNRFSGVPDRFSGSGSGTDFTLKISR VEAEDVGVYYCSQNTHVPPTFGQGTKLEIK |
| Palivizumab(VH) | 3 | QVTLRESGPALVKPTQTLTLTCTFSGFSLSTSGMSVGWIRQ PPGKALEWLADIWWDDKKDYNPSLKSRLTISKDTSKNQV VLKVTNMDPADTATYYCARSMITNWYFDVWGAGTTVTV SS |
| Palivizumab(VL) | 4 | DIQMTQSPSTLSASVGDRVTITCKCQLSVGYMHWYQQKP GKAPKLLIYDTSKLASGVPSRFSGSGSGTEFTLTISSLQPDD FATYYCFQGSGYPFTFGGGTKLEIK |
| Atezolizumab(VH) | 5 | |
| Atezolizumab(VL) | 6 | |
| Elranatamab(VH) | 7 | EVQLLESGGGLVQPGGSLRLSCAASGFTFSSYPMSWVRQA PGKGLEWVSAIGGSGGSLPYADSVKGRFTISRDNSKNTLY LQMNSLRAEDTAVYYCARYWPMDIWGQGTLVTVSS |
| Elranatamab(VL) | 8 | EIVLTQSPGTLSLSPGERATLSCRASQSVSSSYLAWYQQKP GQAPRLLMYDASIRATGIPDRFSGSGSGTDFTLTISRLEPED FAVYYCQQYQSWPLTFGQGTKVEIK |
| Belantamab(VH) | 9 | QVQLVQSGAEVKKPGSSVKVSCKASGGTFSNYWMHWVR QAPGQGLEWMGATYRGHSDTYYNQKFKGRVTITADKSTS TAYMELSSLRSEDTAVYYCARGAIYDGYDVLDNWGQGTL VTVSS |
| Belantamab(VL) | 10 | DIQMTQSPSSLSASVGDRVTITCSASQDISNYLNWYQQKPG KAPKLLIYYTSNLHSGVPSRFSGSGSGTDFTLTISSLQPEDF ATYYCQQYRKLPWTFGQGTKLEIK |
| hRS7(VH) | 11 | SVQLQQSGSELKKPGASVKVSCKASGYTFTNYGMNWVK QAPGQGLKWMGWINTYTGEPTYTDDFKGRFAFSLDTSVS |
| | | TAYLQISSLKADDTAVYFCARGG FGSSYWYFDVWGQGSLVTVSS |
| hRS7(VL) | 12 | DIQLTQSPSSLSASVGDRVSITCKASQDVSIAVAWYQQKPG KAPKLLIYSASYRYTGVPDRFSGSGSGTDFTLTISSLQPEDF AVYYCQQHYITPLTFGAGTKVEIK |
| Linker sequence 1 (GGGGS)₁ | 13 | GGGGS |
| Linker sequence 2 (GGGGS)₂ | 14 | GGGGSGGGGS |
| Linker sequence 3 (GGGGS)₃ | 15 | GGGGSGGGGSGGGGS |
| Linker sequence 4 (GGGGS)₄ | 16 | GGGGSGGGGSGGGGSGGGGS |
| IFNa2b (without signal peptide) | 17 | |
| IFNa2b(L26A) (without signal peptide) | 18 | CDLPQTHSLGSRRTLMLLAQMRRISAFSCLKDRHDFGFPQ EEFGNQFQKAETIPVLHEMIQQIFNLFSTKDSSAAWDETLL DKFYTELYQQLNDLEACVIQGVGVTETPLMKEDSILAVRK YFQRITLYLKEKKYSPCAWEVVRAEIMRSFSLSTNLQESLR SKE |
| IFNa2b(L30A) (without signal peptide) | 19 | CDLPQTHSLGSRRTLMLLAQMRRISLFSCAKDRHDFGFPQ EEFGNQFQKAETIPVLHEMIQQIFNLFSTKDSSAAWDETLL DKFYTELYQQLNDLEACVIQGVGVTETPLMKEDSILAVRK YFQRITLYLKEKKYSPCAWEVVRAEIMRSFSLSTNLQESLR SKE |
| IFNa2b(A145G) (without signal peptide) | 20 | CDLPQTHSLGSRRTLMLLAQMRRISLFSCLKDRHDFGFPQ EEFGNQFQKAETIPVLHEMIQQIFNLFSTKDSSAAWDETLL DKFYTELYQQLNDLEACVIQGVGVTETPLMKEDSILAVRK YFQRITLYLKEKKYSPCAWEVVRGEIMRSFSLSTNLQESLR SKE |
| IFNa2b(R149A) (without signal peptide) | 21 | CDLPQTHSLGSRRTLMLLAQMRRISLFSCLKDRHDFGFPQ EEFGNQFQKAETIPVLHEMIQQIFNLFSTKDSSAAWDETLL DKFYTELYQQLNDLEACVIQGVGVTETPLMKEDSILAVRK YFQRITLYLKEKKYSPCAWEVVRAEIMASFSLSTNLQESLR SKE |
| IFNa2b(S152A) (without signal peptide) | 22 | CDLPQTHSLGSRRTLMLLAQMRRISLFSCLKDRHDFGFPQ EEFGNQFQKAETIPVLHEMIQQIFNLFSTKDSSAAWDETLL DKFYTELYQQLNDLEACVIQGVGVTETPLMKEDSILAVRK YFQRITLYLKEKKYSPCAWEVVRAEIMRSFALSTNLQESLR SKE |
| Signal peptide of chain A and chain B having the first type of structure, signal peptide of first arm (H+L) having the second type of structure | 23 | MRAWIFFLLCLAGRALA |
| Natural signal peptide of IFNa2b, signal peptide of second arm having the second type of structure | 24 | MALTFALLVALLVLSCKSSCSVG |
| Fc | 25 | DKTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKAK GQPREPQVYTLPPSRDELTKNQVSLTCLVKGFYPSDIAVEW ESNGQPENNYKTTPPVLDSDGSFFLYSKLTVDKSRWQQGN VFSCSVMHEALHNHYTQKSLSLSPGK |
| Fc1 | 26 | DKTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKAK GQPREPQVYTLPPSRDELTKNQVSLSCAVKGFYPSDIAVEW ESNGQPENNYKTTPPVLDSDGSFFLVSKLTVDKSRWQQGN VFSCSVMHEALHNHYTQKSLSLSPGK |
| Fc2 | 27 | |
| Fc(N297A) | 28 | DKTHTCPPCPAPELLGGPSVFLFPPKPKDTLMISRTPEVTCV VVDVSHEDPEVKFNWYVDGVEVHNAKTKPREEQYASTY RVVSVLTVLHQDWLNGKEYKCKVSNKALPAPIEKTISKAK GQPREPQVYTLPPSREEMTKNQVSLTCLVKGFYPSDIAVE WESNGQPENNYKTTPPVLDSDGSFFLYSKLTVDKSRWQQ GNVFSCSVMHEALHNHYTQKSLSLSPGK |
| Fc1(N297G) | 29 | DKTHTCPPCPAPELLGGPSVFLFPPKPKDTLMISRTPEVTCV VVDVSHEDPEVKFNWYVDGVEVHNAKTKPREEQYGSTY RVVSVLTVLHQDWLNGKEYKCKVSNKALPAPIEKTISKAK GQPREPQVYTLPPSRDELTKNQVSLSCAVKGFYPSDIAVEW ESNGQPENNYKTTPPVLDSDGSFFLVSKLTVDKSRWQQGN VFSCSVMHEALHNHYTQKSLSLSPGK |
| Fc2(N297G) | 30 | DKTHTCPPCPAPELLGGPSVFLFPPKPKDTLMISRTPEVTCV VVDVSHEDPEVKFNWYVDGVEVHNAKTKPREEQYGSTY RVVSVLTVLHQDWLNGKEYKCKVSNKALPAPIEKTISKAK |
| | | GQPREPQVYTLPPSRDELTKNQVSLWCLVKGFYPSDIAVE WESNGQPENNYKTTPPVLDSDGSFFLYSKLTVDKSRWQQ GNVFSCSVMHEALHNHYTQKSLSLSPGK |
| CH1 | 31 | ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSC |
| CL | 32 | |
| GC33(H)-Fc | 33 | QVQLVQSGAEVKKPGASVKVSCKASGYTFTDYEMHWVR QAPGQGLEWMGALDPKTGDTAYSQKFKGRVTLTADKSTS TAYMELSSLTSEDTAVYYCTRFYSYTYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCDKTHTCPPCPAPELLGGPSVFL FPPKPKDTLMISRTPEVTCVVVDVSHEDPEVKFNWYVDGV EVHNAKTKPREEQYNSTYRVVSVLTVLHQDWLNGKEYKC KVSNKALPAPIEKTISKAKGQPREPQVYTLPPSREEMTKNQ VSLTCLVKGFYPSDIAVEWESNGQPENNYKTTPPVLDSDGS FFLYSKLTVDKSRWQQGNVFSCSVMHEALHNHYTQKSLS LSPGK |

### Example 2. Plasmid construction, expression, and purification of bispecific recombinant protein

### 1. Plasmid construction, cell transfection, and protein expression

The bispecific recombinant protein expression plasmid was synthesized by GENEWIZ after codon optimization based on the protein sequence, ligated into the pCDNA3.1 plasmid, and the sequence was verified by sequencing.

After the obtained expression plasmid was filtered through a 0.22 µm filtration membrane, 50 µg of the plasmid solution (wherein the product was a bispecific recombinant protein having the first type of structure, and the mass ratio of the chain A and the chain B expression plasmids was 2:1 or 3:1; wherein the product was a bispecific recombinant protein having the second type of structure, and the mass ratio of the light chain, the heavy chain, and the second arm expression plasmids was 3:2:2) was pipetted into 2 mL of OptiPRO SFM Medium (GIBCO) and mixed thoroughly. 160 µL of transfection reagent (ExpiFectamine CHO Reagent) was pipetted into 2 mL of OptiPRO SFM Medium and mixed thoroughly. The obtained mixed solution of transfection reagent was added to the mixed solution containing the plasmid and mixed thoroughly. The mixture of the plasmid and the transfection reagent was slowly and evenly added into 50 mL of a suspension of host cell ExpiCHO-S (Thermo Fisher) with a cell density of 6×10⁶ viable cells/mL, and then cultured in an incubator containing 8% CO₂ at 37°C on the first day (after 18 to 22 hours), 300 µL of ExpiCHO Enhancer and 8 mL of ExpiCHO Feed were added therein, and the culture temperature was lowered to 32°C; on the fifth day, a second feeding was performed with 8 mL of ExpiCHO Feed, and the cell suspension was harvested upon 12 days of culture. The cell suspension was centrifuged at 8,000 rpm for 15 minutes, and the supernatant obtained by centrifugation, (i.e., the cell culture harvest solution) was used for purification of the target protein.

### 2. Protein purification

### 2.1. Sample capture

The expression supernatant of bispecific recombinant protein was used to capture the target protein using MabSelect SuRe (i.e., protein A, purchased from Cytiva) affinity resin. The experimental procedures are as follows:
a) equilibration: an equilibration buffer (50 mM Tris-HCl, 150 mM NaCl, pH 7.2) was used to equilibrate the chromatography column until the UV detection line was stable;
b) loading: the sample was loaded using a sample pump with a retention time of 5 minutes and a loading capacity of 30 mg/mL or less;
c) re-equilibration: 5 column volumes of the equilibration buffer (50 mM Tris-HCl, 150 mM NaCl, pH 7.2) was used to wash the chromatography column;
d) elution: an eluate (50 mM NaAC-HAC, pH 3.5) was used to elute the target protein, then the pH of the eluate was adjusted to 5.5 with 1 M Tris buffer, and the purity of the protein was detected by SDS-PAGE.

### 2.2. Sample secondary purification

### 2.2.1 This example takes TTM101-LC01 as an example to describe the secondary purification procedure of the bispecific recombinant protein having the first type of structure.

The expression supernatant of bispecific recombinant protein TTM101-LC01 was used to remove aggregates and other impurities in the sample using SULFATE 650F resin (TOSOH). The experimental procedures are as follows:
a) equilibration: an equilibration buffer (50 mM NaAC-HAC, pH 5.5) was used to equilibrate the chromatography column until the UV detection line was stable;
b) loading: the sample was loaded using a sample pump with a retention time of 5 minutes and a loading capacity of 50 mg/mL or less;
c) re-equilibration: 5 column volumes of the equilibration buffer (50 mM NaAC-HAC, pH 5.5) was used to wash the chromatography column;
d) elution: an eluate (50 mM NaAC-HAC, 250 mM, pH 5.5) was used to elute the target protein, and the purity of the protein was detected by SDS-PAGE.

### 2.2.2 This example takes TTM101-01 as an example to describe the secondary purification procedure of the bispecific recombinant protein having the second type of structure.

The expression supernatant of bispecific recombinant protein TTM101-01 was used to remove impurities using KappaSelect affinity resin (Cytiva) in the sample. The experimental procedures are as follows:
a) equilibration: an equilibration buffer (50 mM Tris-HCl, 150 mM NaCl, pH 7.2) was used to equilibrate thechromatography column until the UV detection line was stable;
b) loading: the sample was loaded using a sample pump with a retention time of 5 minutes and a loading capacity of 30 mg/mL or less;
c) re-equilibration: 5 column volumes of the equilibration buffer (50 mM Tris-HCl, 150 mM NaCl, pH 7.2) was used to wash the chromatography column;
d) elution: an eluate (0.1 M Gly-HCl, pH 2.7) was used to elute the target protein, then the pH of the eluate was adjusted to 5.5 with 1 M Tris buffer, and the purity of the protein was detected by SDS-PAGE.

The purification methods of the control samples Codrituzumab, IFN-α 2b-Fc, and Atezolizumab (listed in Table 3) are the same as described in 2.1.

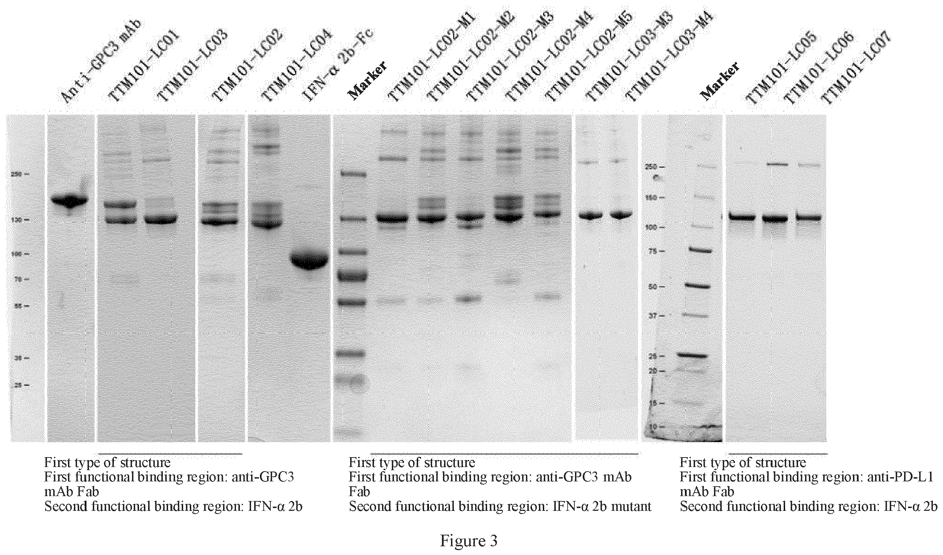

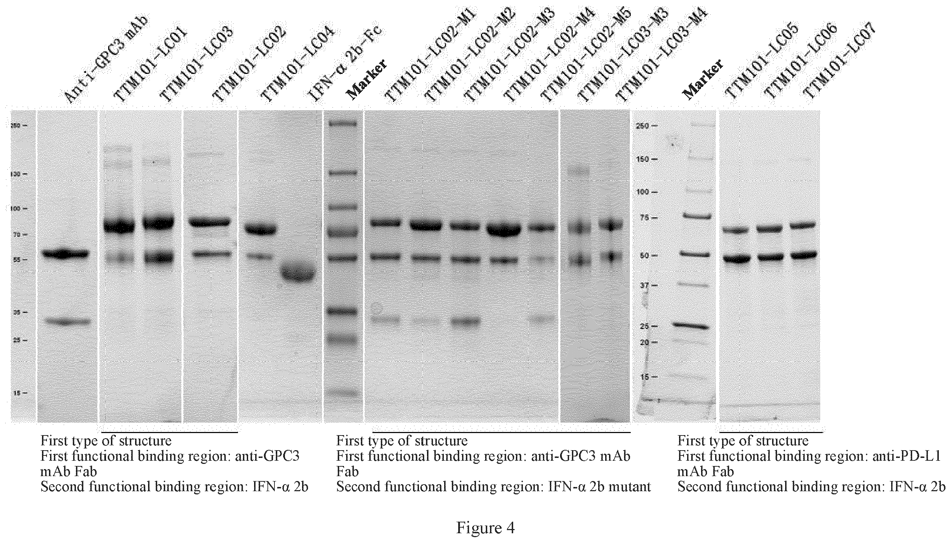

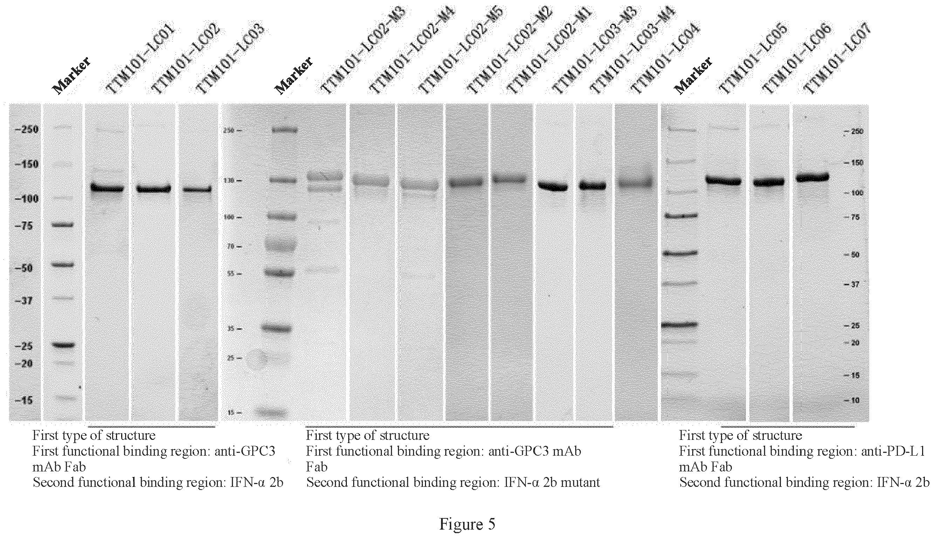

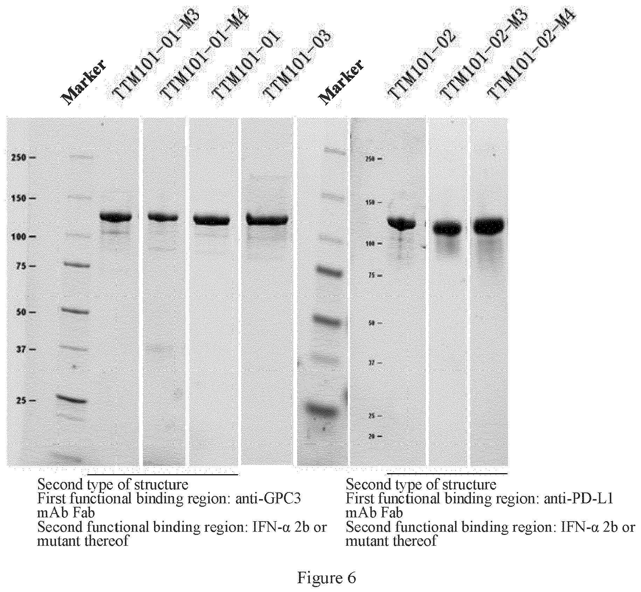

The theoretical molecular weights of the bispecific recombinant proteins (listed in Tables 1 and 2) are all approximately 120 kD, and the molecular weights of the control antibodies Codrituzumab, IFNα2b-Fc, and IFNα2b monomer (listed in Table 3) are 150 KD, 88 KD, and 19.2 KD, respectively. The non-reducing SDS-PAGE protein electrophoresis results of the affinity captured samples of the bispecific recombinant protein having the first type of structure (i.e., after purification of protein A) are shown in Figure 3, and the reducing SDS-PAGE protein electrophoresis results are shown in Figure 4. The electrophoresis results show that the antibody expressed by this structure has a high content of aggregates. The non-reducing SDS-PAGE protein electrophoresis results after secondary purification (i.e., after SULFATE 650F purification) are shown in Figure 5. It can be seen that most of the aggregates can be removed by cation exchange chromatography resin. The non-reducing SDS-PAGE protein electrophoresis results of the bispecific recombinant protein having the second type of structure (see Figure 2) after secondary purification (i.e., after Kappa purification) are shown in Figure 6. The results show that bispecific recombinant proteins with high purity can be obtained by protein A affinity purification and Kappa affinity purification.

### Example 3. Detection of binding affinity of GPC3-targeted bispecific recombinant proteins to GPC3-positive liver cancer cell lines (double-positive expressing cells, target cells)

The binding affinity of the GPC3-targeted bispecific recombinant protein to the target GPC3-positive liver cancer cell line was determined by flow cytometry. The following method takes TTM101-LC01, TTM101-LC02, TTM101-LC03, and TTM101-01 as examples, and is suitable for the assay of the bispecific recombinant protein whose first functional binding region binds to GPC3 antigen and whose second functional binding region is IFN-α 2b or a low-affinity mutant thereof.

The cells for the assay were HepG2 cells (Nanjing Kebai Biotechnology Co., Ltd.) and HuH-7 cells (Cell Bank of Chinese Academy of Sciences, Shanghai). Well-grown cells were collected and counted, centrifuged and resuspended with FACS buffer (PBS+2% FBS) to a concentration of 1×10⁶ cells/mL. The cells were added to a 96-well U-shaped plate (Cat. No.: 3799, Corning) at 100 µL/well, then 7 dilutions of bispecific recombinant proteins or control proteins (3-fold serial dilutions starting from 100 nM, a total of 7 concentrations) were added therein, respectively, and the plate was incubated at 4°C for 1 hour; the cells were washed with FACS buffer, then goat anti-human IgG (Alexa Fluor488 goat anti-human IgG (H+L), Invitrogen) was added therein, and the plate was incubated at 4°C for 1 hour; the cells were rinsed and resuspended with FACS buffer, and the fluorescence value was determined by flow cytometry (Attune Nxt, Invitrogen).

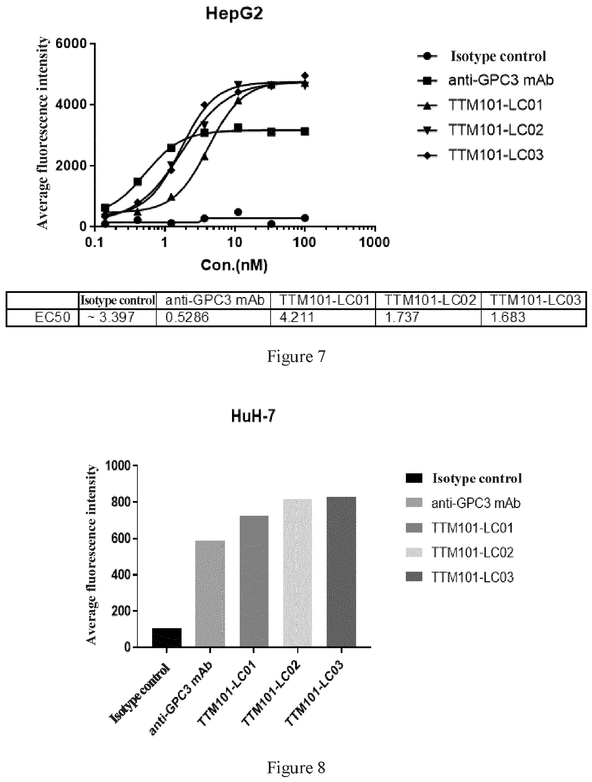

The experimental results are shown in Figure 7 and Figure 8. The bispecific recombinant proteins having the first type of structure (TTM101-LC01, TTM101-LC02, and TTM101-LC03) all have certain binding affinity to the target cells (HepG2 and HuH-7 cells) that express GPC3 and IFNα receptors simultaneously, and the bispecific recombinant protein binding to HepG2 and HuH-7 cells has a higher maximum average fluorescence intensity than that of anti-GPC3 monoclonal antibody (anti-GPC3 mAb, i.e., control sample Codrituzumab). At the same time, as shown in Figure 7 and Figure 8, the length of the linker sequence has a weak influence on the binding affinity of the bispecific recombinant protein to the target cells. When the linker sequence is one GGGGS, the binding affinity (EC₅₀) of the recombinant protein is relatively lowest.

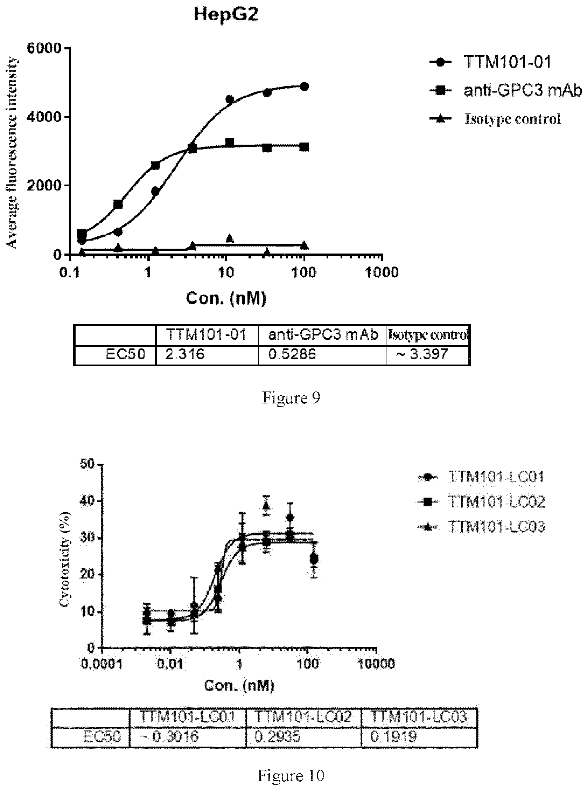

As shown in Figure 9, the bispecific recombinant protein having the second type of structure (TTM101-01) hascertain binding affinity to the target cells (HepG2 cells) that express GPC3 and IFN receptors simultaneously, the EC₅₀ is comparable to that of anti-GPC3 monoclonal antibody (anti-GPC3 mAb, i.e., control sample Codrituzumab), and the bispecific recombinant protein (TTM101-01) has a higher maximum average fluorescence intensity than that of anti-GPC3 monoclonal antibody (anti-GPC3 mAb, i.e., control sample Codrituzumab).

### Example 4. Detection of the antibody-dependent cell-mediated cytotoxicity (ADCC) activity GPC3-targeted bispecific recombinant protein

The ADCC activity of the bispecific recombinant protein on the target GPC3-positive liver cancer cell line was determined by the lactate dehydrogenase (LDH) method. The following method takes TTM101-LC01, TTM101-LC02, TTM101-LC03, and TTM101-01 as examples, and is suitable for the detection of the ADCC activity of the bispecific recombinant protein, whose first functional binding region binds to GPC3 antigen and whose second functional binding region is IFN-α 2b or a low-affinity mutant thereof.

Human peripheral blood mononuclear cells (PBMC) were used as effector cells, GPC3-positive HepG2 liver cancer cell lines were used as target cells, and the LDH assay kit was CytoTox-ONE^{™} Homogeneous Membrance Integrity Assay (Promega, G7892). The target cells and effector cells were plated at a ratio of 1:20, then added with bispecific recombinant proteins or control proteins (5-fold serial dilutions starting from 150 nM, a total of 8 concentrations), and incubated at 37°C for 4 hours. After incubation at 37°C for 3.5 hours, lysis reagent was added to the control group, and the cells were observed under a microscope. After the cells were completely lysed, the lysate was centrifuged at 10,000 rpm for 5 minutes. The supernatant was transferred to a 96-well black-bottomed transparent plate (Corning, 3904), and incubated with the substrate at 37°C for 30 minutes. Stop solution was then added therein, and the plate was shaken for 3 to 5 minutes in dark. The signal value was detected by a microplate reader (SpectraMax M2). For details, refer to the LDH assay kit instructions.

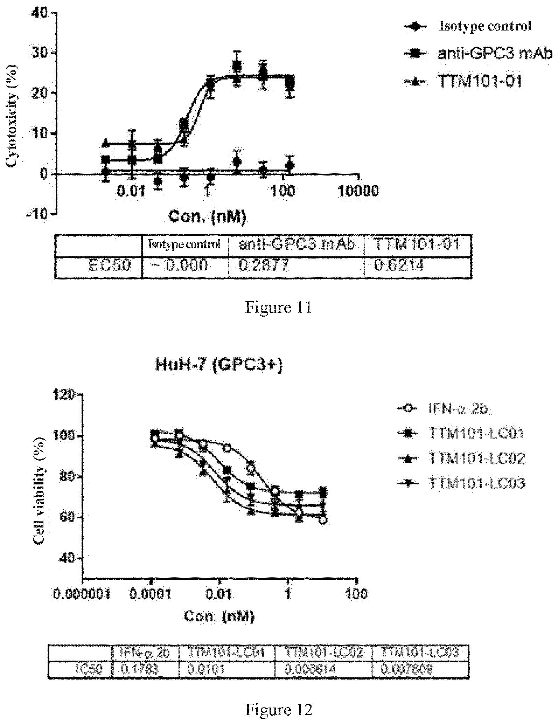

As shown in Figure 10 and Figure 11, the bispecific recombinant protein having the first type of structure (e.g., TTM101-LC01, TTM101-LC02, or TTM101-LC03) and the bispecific recombinant protein having the second type of structure (e.g., TTM101-01) retain ADCC activity, and the ADCC activity of the bispecific recombinant protein is comparable to that of anti-GPC3 monoclonal antibody (anti-GPC3 mAb, i.e., control sample Codrituzumab) (see Figure 11).

### Example 5. Detection of proliferation inhibitory activity of GPC3-targeted bispecific recombinant protein

The proliferation inhibitory activity of the GPC3-targeted bispecific recombinant protein on different tumor cell lines was determined by cell titer glo kit (Promega, Cat: G7558). The following method takes TTM101-LC01, TTM101-LC02, and TTM101-LC03 as examples, and is suitable for the detection of the bispecific recombinant protein whose first functional binding region binds to GPC3 antigen and whose second functional binding region is IFNα2b or a low-affinity mutant thereof.

GPC3-positive cell line HuH-7 or GPC3-negative tumor cell line U266 (purchased from Nanjing Kebai Biotechnology Co., Ltd.) and GPC3-negative tumor cell line SW480 (purchased from Nanjing Kebai Biotechnology Co., Ltd.) were plated in a 96-well black-bottomed transparent plate (Corning, 3904), then bispecific recombinant proteins or control proteins (10-fold serial dilutions, starting from 52 nM, a total of 6 points; or 5-fold serial dilutions starting from 10 nM, a total of 8 points) were added therein, and the plate was incubated in a CO₂ incubator at 37°C for 3 days. Cell titer glo was added therein, and the signal value was detected by Multomode Plate Reader (PerkinElmer, Envision2105).

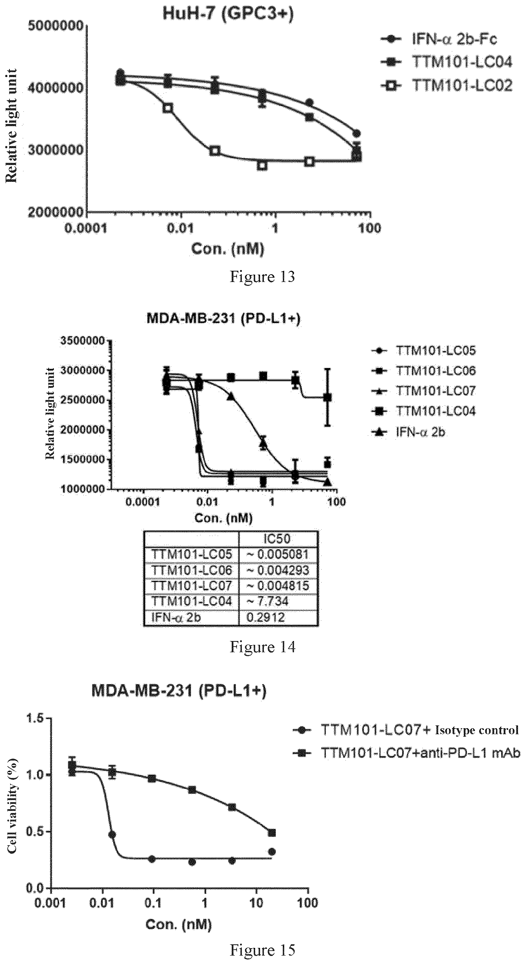

The results show that the proliferation inhibitory activity (IC₅₀) of the bispecific recombinant protein (e.g., TTM101-LC01, TTM101-LC02, or TTM101-LC03) on GPC3-positive (GPC3+) target cells HuH-7 is higher than that of the control IFN-α 2b (see Figure 12), and is also higher than that of anti-GPC3 monoclonal antibody (anti-GPC3 mAb, i.e., control sample Codrituzumab, which has no proliferation inhibitory effect on HuH-7). As shown in Figure 13, the proliferation inhibitory activity of the bispecific recombinant protein TTM101-LC04 lacking GPC3 targeting function and the Fc fusion protein of IFN-α 2b on HuH-7 (GPC3-positive target cells) is significantly lower than that of the bispecific recombinant protein TTM101-LC02 with GPC3 targeting function, indicating that the bispecific recombinant protein with GPC3 targeting function binding to GPC3 on target cells can significantly enhance the proliferation inhibitory activity of IFN-α 2b, while the bispecific recombinant protein without the effect of targeting the target cells has a low proliferation inhibitory activity of IFN-α2b. It reveals that the bispecific recombinant protein of the present disclosure has a strong proliferation inhibitory effect only on the target cells with the target antigen, but has a weak effect on or does not bind to the non-target cells without the target antigen, indicating that the bispecific recombinant protein of the present disclosure has a high safety profile.

In addition, as shown in Table 5, the bispecific recombinant proteins having the first type of structure with different linker sequences (taking TTM101-LC01, TTM101-LC02, and TTM101-LC03 as examples) have stronger proliferation inhibitory activity on GPC3-positive (GPC3+) target cells (taking HuH-7 as an example) than that of IFN-α 2b, and the bispecific recombinant proteins with different linker sequences have a slight difference in proliferation inhibitory activity. The bispecific recombinant protein containing one GGGGS as the linker sequence (i.e. the bispecific recombinant protein linked to a short linker sequence, taking TTM101-LC01 as an example) has relatively low activity (see Figure 12 and Table 5), which is consistent with the binding affinity of the recombinant proteins with different linker sequences as shown in Figure 8. In GPC3-negative (GPC3-) non-target cells U266 and SW480 cell lines, the bispecific recombinant proteins (taking TTM101-LC01, TTM101-LC02, and TTM101-LC03 as examples) have significantly lower proliferation inhibitory activity than that of IFN-α 2b (i.e. the relative activity of the bispecific recombinant protein is much less than 1), which indicates that in cells that do not express GPC3 (i.e. non-target cells that do not express the target antigen), the bispecific recombinant protein has extremely low proliferation inhibitory activity, suggesting it has a high safety profile. At the same time, as shown in Table 5, the proliferation inhibitory activity of the bispecific recombinant protein on target cells is at least 700 times higher than that of the bispecific recombinant protein on non-target cells.

**Table 5: Relative proliferation inhibitory activities of bispecific recombinant proteins with different linker sequences**

| Sample | Relative activity (IC₅₀ IFN-α 2b/bispecific recombinant protein) | | | Relative activity (IC₅₀ non-target cell/target cell) | |
|---|---|---|---|---|---|
| | HuH-7 (GPC3+) | U266 (GPC3-) | SW480 (GPC3-) | U266 (GPC3-)/HuH-7 (GPC3+) | SW480 (GPC3-)/HuH-7 (GPC3+) |
| TM101-LC01 | 17.80 | 0.008 | 0.023 | 2225.000 | 773.913 |
| TTM101-LC02 | 27.00 | 0.034 | 0.017 | 794.118 | 1588.235 |
| TTM101-LC03 | 23.40 | 0.004 | 0.030 | 5850.000 | 780.000 |

### Example 6. Detection of proliferation inhibitory activity of PD-L1-targeted bispecific recombinant protein

The proliferation inhibitory activity of the PD-L1-targeted bispecific recombinant protein on different tumor cell lines was determined by cell titer glo kit (Promega, Cat: G7558). The following method takes TTM101-LC05, TTM101-LC06, and TTM101-LC07 as examples, and is suitable for the bispecific recombinant protein whose first functional binding region binds to PD-L1 antigen and whose second functional binding region is IFNα 2b or a low-affinity mutant thereof.

PD-L1-positive cell line MDA-MB-231 (purchased from Nanjing Kebai Biotechnology Co., Ltd.) (human breast cancer cells) was plated in a 96-well black-bottomed transparent plate (Corning, 3904), then bispecific recombinant proteins or control proteins (10-fold serial dilutions starting from 52 nM, a total of 6 concentrations) were added therein, and the plate was incubated in a CO₂ incubator at 37°C for 3 days. Cell titer glo was added therein, and the signal value was detected by Multomode Plate Reader (PerkinElmer, Envision2105).

As shown in Figure 14, for PD-L1-positive (PD-L1+) target cells MDA-MB-231, PD-L1-targeted bispecific recombinant proteins (TTM101-LC05, TTM101-LC06, and TTM101-LC07) all have proliferation inhibitory activity, and the activity is significantly higher than that of the control bispecific recombinant protein TTM101-LC04 without the function of targeting PD-L1 and IFN-α 2b, indicating that targeting PD-L1 can significantly enhance the proliferation inhibitory activity of IFN-α 2b. It shows that the bispecific recombinant protein with PD-L1 targeting function binding to PD-L1 on target cells MDA-MB-231 can significantly enhance the proliferation inhibitory activity of IFN-α 2b, while the bispecific recombinant protein without the effect of targeting the target cells has a low proliferation inhibitory activity of IFN-α2b. It reveals that the bispecific recombinant protein of the present disclosure has a strong proliferation inhibitory effect only on the target cells with the target antigen, but has a weak effect on or does not bind to the non-target cells that do not express the target antigen, indicating that the bispecific recombinant protein of the present disclosure has a high safety profile.

PD-L1-positive target cells MDA-MB-231 were plated in a 96-well black-bottomed transparent plate (Corning, 3904), then 200 nM PD-L1 antibody Atezolizumab or isotype control was added therein, and the plate was incubated at 37°C for 30 minutes. Bispecific recombinant proteins or control proteins (6-fold serial dilutions starting from 20 nM, a total of 6 concentration points) were added therein, and the plate was incubated in a CO₂ incubator at 37°C for 3 days. Cell titer glo was added therein, and the signal value was detected by Multomode Plate Reader (PerkinElmer, Envision2105).

As shown in Figure 15, after Atezolizumab was added to block the binding site of PD-L1 antigen and antibody, the proliferation inhibitory activity of TTM101-LC07 was significantly reduced. The results prove that blocking the binding function of the target antigen on the cells to be tested results in a significant decrease in the proliferation inhibitory activity of the bispecific recombinant protein on the cells to be tested, which demonstrates from another perspective that the bispecific recombinant protein of the present disclosure has a weak effect on or does not bind to non-target cells without the ability to bind to the target antigen, indicating that the bispecific recombinant protein of the present disclosure has a high safety profile.

### Example 7. Detection of proliferation inhibitory activity of bispecific recombinant protein containing IFN-α 2b low-affinity mutant

Considering the difference between the tolerance dose of human body to IFN-α 2b and the effective dose of conventional antibodies, in order to better match the effect of the target antigen-targeted half-antibody and IFN-α 2b to achieve high efficiency and low toxicity, the present disclosure also designed a series of bispecific recombinant proteins containing IFN-α 2b low-affinity mutants. This example takes the mutation design based on TTM101-LC02 as an example to design the bispecific recombinant proteins containing IFN-α 2b low-affinity mutants, and the relative affinity of the mutants based on the mutation site of wild-type IFN-α 2b to IFNAR2 (IFNα receptor 2) (data refer to Cell. 2011 August 19; 146(4): 621-632.) as shown in Table 6 to detect the proliferation inhibitory activity of the bispecific recombinant proteins containing different IFN-α 2b low-affinity mutants. The experimental method is the same as described in Example 5.

**Table 6: Mutation site of IFN-α 2b and relative affinity of IFNAR2**

| Mutation site of IFN-α 2b | Relative affinity of IFNAR2 |
|---|---|
| L26A | 0.22 |
| L30A | 0.0013 |
| A145G | 0.03 |
| R149A | 0.01 |
| S152A | 0.1 |

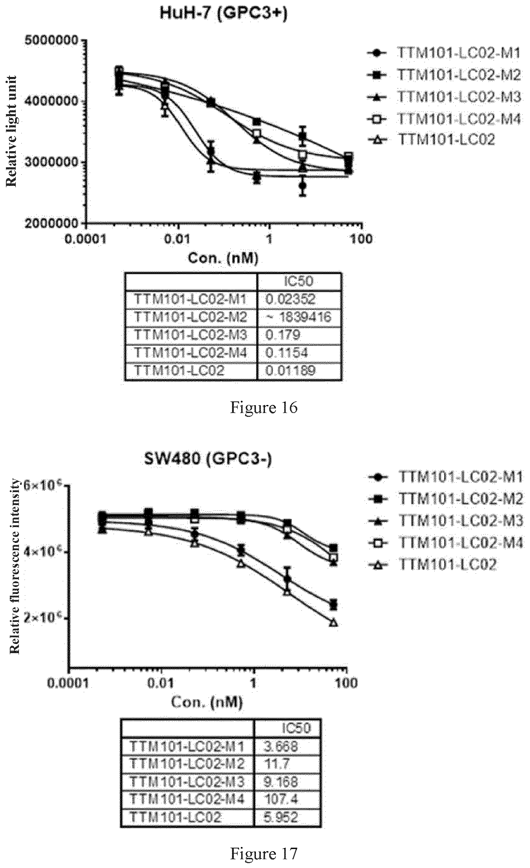

As shown in Figures 16 to 18, the results show that the proliferation inhibitory activities of the bispecific recombinant proteins (TTM101-LC02-M2, TTM101-LC02-M3, and TTM101-LC02-M4) containing IFN-α 2b low-affinity mutants on GPC3-positive target cells (HuH-7) or GPC3-negative non-target cells (U266 and SW480) are all reduced compared to TTM101-LC02. For example, the proliferation inhibitory activity IC₅₀ of TTM101-LC02-M3 on HuH-7 (GPC3-positive cell line, target cell) is comparable to that of IFN-α 2b (IC_{50 IFN-α 2b} = 0.1793, IC_{50 TTM101-LC02-M3} = 0.179), but the relative proliferation inhibitory activity (IC₅₀ IFN-α 2b/bispecific recombinant protein) of TTM101-LC02-M3 on GPC3-negative non-target cells (U266) is weaker, for example, the relative proliferation inhibitory activity (IC₅₀ IFN-α 2b/bispecific recombinant protein) of TTM101-LC02-M3 on U266 is 0.000615, which is significantly weaker than that of IFN-α 2b and weaker than that of TTM101-LC02. TTM101-LC02-M2, TTM101-LC02-M4, TTM101-LC03-M3, and TTM101-LC03-M4 also have similar results.

The above results show that the activity of the GPC3-targeted bispecific recombinant proteins having the first type of structure containing IFN-α 2b low-affinity mutants on the target antigen-positive target cell line is comparable to that of IFN-α 2b, but the activity decreases on the non-target cell line that does not express the target antigen. It shows that the A145G or R149A mutation has good targeting ability and high safety profile in the GPC3- or PD-L1-targeted bispecific recombinant protein having the first type of structure.

In order to further verify the targeting of the first type of structure, the bispecific recombinant proteins targeting Trop-2, BCMA, and PD-L1 were detected for differences in their activity on target-negative and target-positive tumor cells, respectively. The experimental methods are the same as described in Examples 5 and 6.

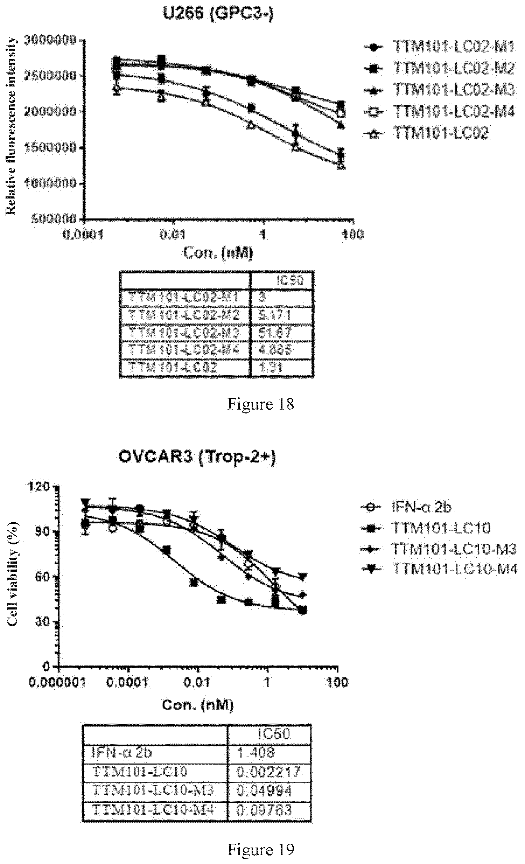

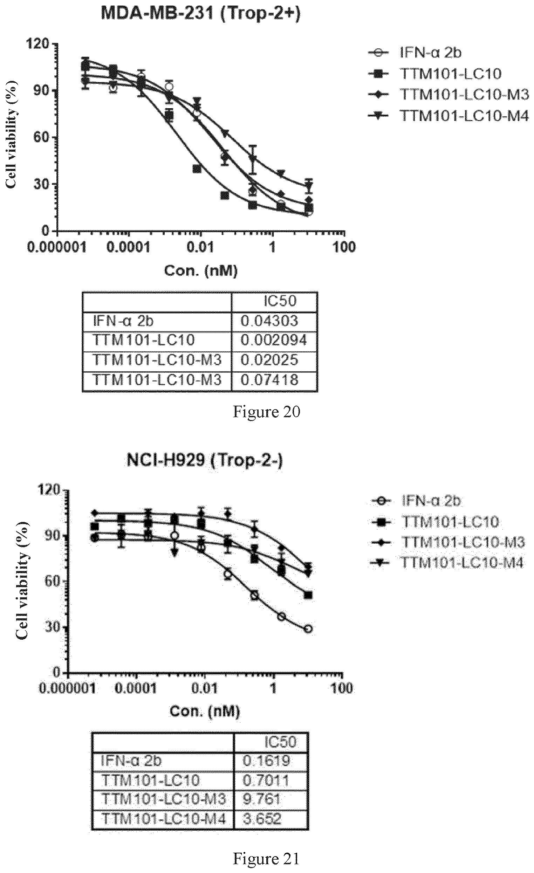

As shown in Figures 19 to 21, the bispecific recombinant proteins containing IFN-α 2b low-affinity mutants were detected for their activity on Trop-2-positive target cells (OVCAR3 and MDA-MB-231) and Trop-2-negative target cells (NCI-H929). The results show that the proliferation inhibitory activities of TTM101-LC10-M3 and TTM101-LC10-M4 on Trop-2-negative and Trop-2-positive cells are reduced compared to TTM101-LC10. However, on Trop-2-positive target cells (OVCAR3 and MDA-MB-231), the proliferation inhibitory activities of TTM101-LC10, TTM101-LC10-M3, and TTM101-LC10-M4 are superior or comparable to that of IFN-α 2b; on Trop-2-negative target cells (NCI-H929), the proliferation inhibitory activities of TTM101-LC10, TTM101-LC10-M3, and TTM101-LC10-M4 are significantly reduced compared to IFN-α 2b, especially the activities of TTM101-LC10-M3 and TTM101-LC10-M4 are reduced by more than 20 times compared to IFN-α 2b. The above results show that the A145G or R149A mutation has good targeting ability and high safety profile in the Trop-2-targeted bispecific recombinant protein having the first type of structure.

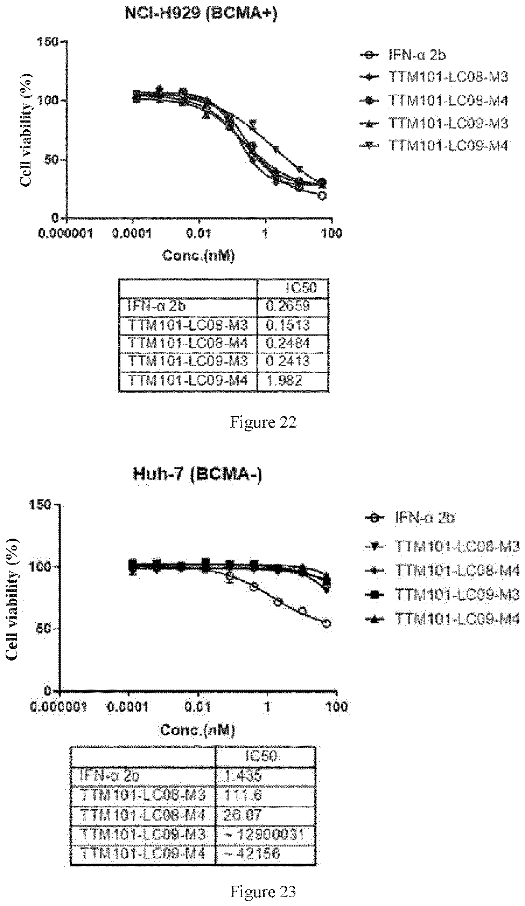

As shown in Figures 22 to 24, the bispecific recombinant proteins containing IFN-α 2b low-affinity mutants were detected for their activity on BCMA-positive target cells (NCI-H929). The results show that the proliferation inhibitory activities of TTM101-LC08-M3, TTM101-LC09-M3, and TTM101-LC08-M4 are comparable to that of IFN-α 2b, and the activities of TTM101-LC09-M4 are approximately 7 times weaker than that of IFN-α 2b. On BCMA-negative non-target cells (HuH-7 and MDA-MB-231), the proliferation inhibitory activities of TTM101-LC08-M3, TTM101-LC09-M3, TTM101-LC08-M4, and TTM101-LC09-M4 are significantly lower than that of IFN-α 2b. It shows that the A145G or R149A mutation has good targeting ability and high safety profile in the BCMA-targeted bispecific recombinant protein having the first type of structure.

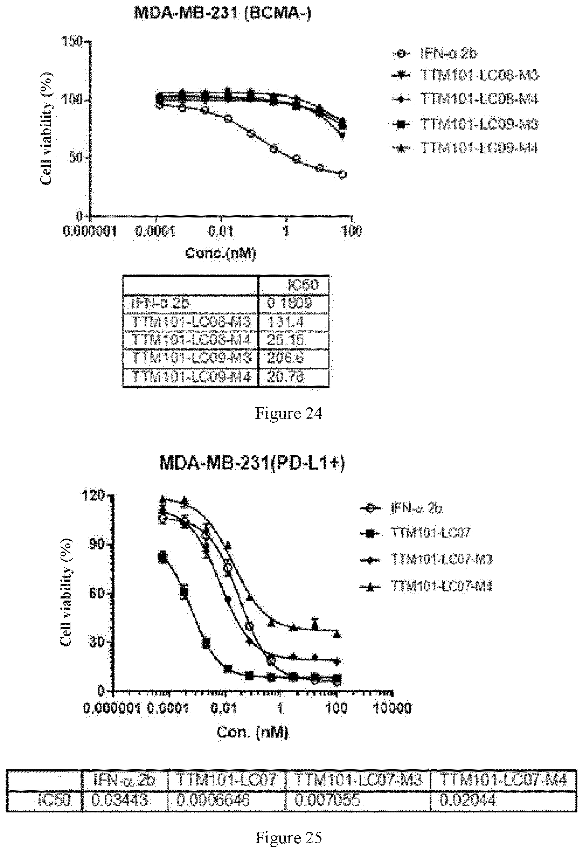

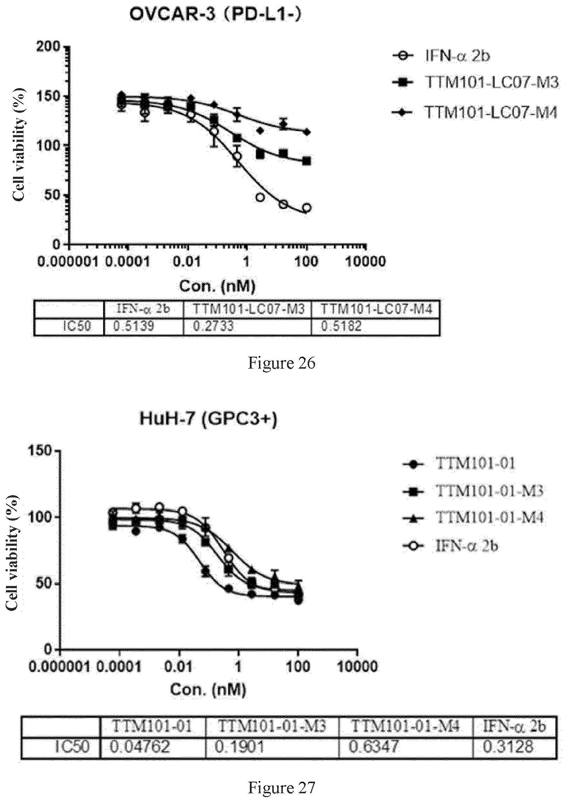

As shown in Figure 25 and Figure 26, the bispecific recombinant proteins containing IFN-α 2b low-affinity mutants were detected for their activity on PD-L1-positive target cells (MDA-MB-231) and PD-L1-negative target cells (OVCAR3). The results show that the proliferation inhibitory activities of TTM101-LC07-M3 and TTM101-LC07-M4 on PD-L1-positive cells are reduced compared to TTM101-LC07. The proliferation inhibitory activities of TTM101-LC07 and TTM101-LC07-M3 are approximately 5 times higher than that of IFN-α 2b, and the activity of TTM101-LC07-M4 is comparable to that of IFN-α 2b, but the proliferation inhibitory activities of TTM101-LC07-M3 and TTM101-LC07-M4 on PD-L1-negative cells (OVCAR3) are significantly weaker than that of IFN-α 2b, indicating that the A145G or R149A mutation has good targeting ability and high safety profile in the PD-L1-targeted bispecific recombinant protein having the first type of structure.

At the same time, taking the A145G and R149A mutations as examples, the inventors constructed the GPC3- and PD-L1-targeted bispecific recombinant proteins having the second type of structure.

This example takes TTM101-01-M3 and TTM101-01-M4 as examples to illustrate that the GPC3-targeted bispecific recombinant proteins having the second type of structure containing IFN-α 2b low-affinity mutants were detected for their proliferation inhibitory activity on GPC3-positive target cells (HuH-7) and GPC3-negative non-target cells (SW480, MDA-MB-231, and U266), respectively. The experimental method is the same as described in Example 5. The proteins to be tested and control proteins were subjected to a 5-fold serial dilutions starting from 100 nM with a total of 9 dilutions.

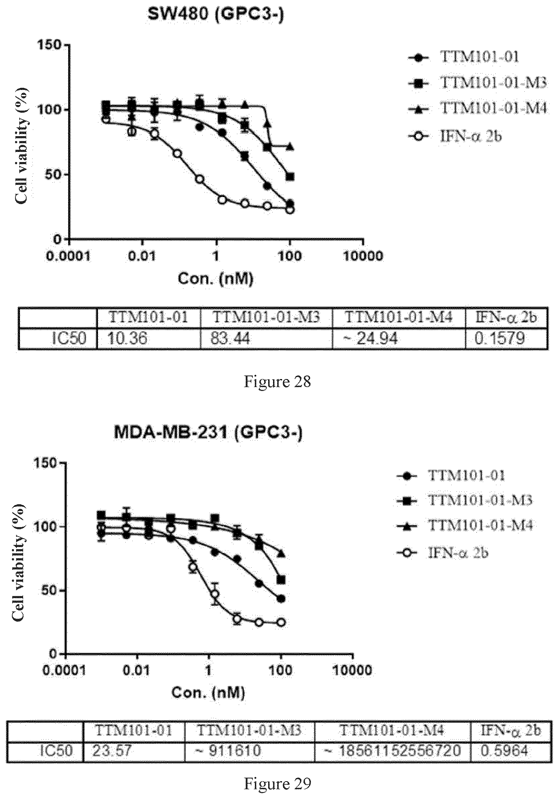

The results show that on GPC3-positive target cells (HuH-7, target cells) and GPC3-negative non-target cells (U266, MDA-MB-231, or SW480, non-target cells), the proliferation inhibitory activities of TTM101-01-M3 and TTM101-01-M4 are reduced compared to TTM101-01, but there is a significant difference in the degree of reduction between target cells and non-target cells. Among them, the proliferation inhibitory activities IC₅₀ of TTM101-01-M3 and TTM101-01-M4 on GPC3-positive target cells (HuH-7) are basically comparable to that of IFN-α 2b (Figure 27); whereas on GPC3-negative cell lines (i.e., non-target cells), the proliferation inhibitory activities of TTM101-01-M3 and TTM101-01-M4 are more than 100 times lower than that of IFN-α 2b (Figures 28 to 30), especially their proliferation inhibitory activities on U266 and MDA-MB-231 are more than 10,000 times lower than that of IFN-α 2b.

The above results show that the activity of the GPC3- or PD-L1-targeted bispecific recombinant protein having the second type of structure containing IFN-α 2b low-affinity mutants with the A145G or R149A mutation on the target antigen-positive target cell line is comparable to that of IFN-α 2b, but the activity on the non-target cell line that does not express the target antigen is significantly reduced, and the activity of the R149A mutation is relatively lower. It shows that the A145G or R149A mutation has good targeting ability and high safety profile in the GPC3- or PD-L1-targeted bispecific recombinant protein having the second type of structure.

In summary, the degree of the reduction in the proliferation inhibitory activity of the bispecific recombinant protein of the present disclosure containing IFN-α 2b low-affinity mutants on target antigen-negative non-target cells relative to the proliferation inhibitory activity of the bispecific recombinant protein containing wild-type IFN-α 2b on target antigen-negative non-target cells is comparable to or more significant than the degree of the reduction in the proliferation inhibitory activity of the bispecific recombinant protein containing IFN-α 2b low-affinity mutants on target antigen-positive target cells.

### Example 8. Detection of proliferation inhibitory activity of potentially risky impurities of bispecific recombinant protein on non-target cells

In order to reduce the potential safety risk of impurities in the future preparation process of the bispecific recombinant protein, the present disclosure takes TTM101-LC02 and TTM101-01 as examples to analyze the proliferation inhibitory effect of potentially risky impurities (chain B or second arm homodimers) on non-target cells.

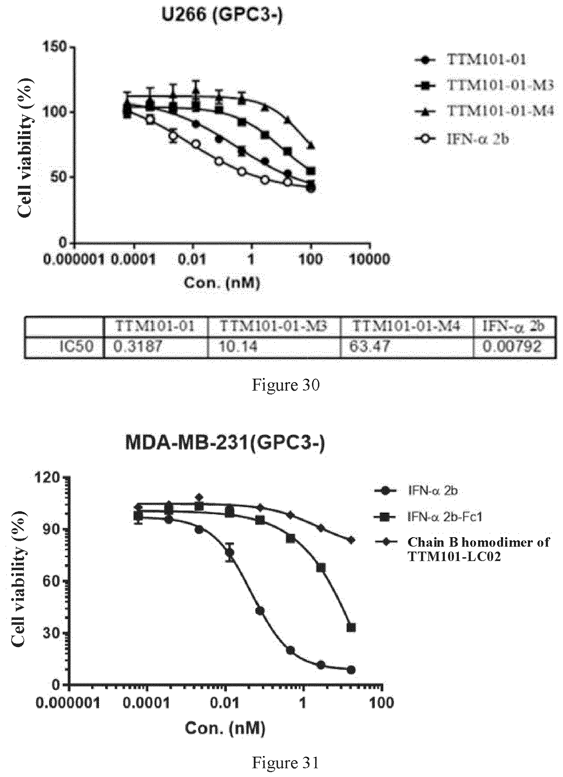

In the secondary purification process of TTM101-LC02 (as described in Example 2), the chain B homodimer of TTM101-LC02 (Figure 3, the molecular weight of the homodimer is approximately 140 kD) was isolated, and the proliferation inhibitory activity of IFN-α 2b-Fc (the second arm homodimer of TTM101-01) on non-target cells (GPC3-negative cells) MDA-MB-231 was detected. As shown in Figure 31, at a concentration of 16.7 nM, the proliferation inhibitory rate of IFN-α 2b on MDA-MB-231 (GPC3-negative cells, non-target cells) is 91.3%, the proliferation inhibitory rate of the second arm homodimer of TTM101-01 (IFN-α 2b-Fc) on MDA-MB-231 (GPC3-negative cells, non-target cells) is 66.8% (reduced by approximately 24.5% compared to IFN-α 2b), and the proliferation inhibitory rate of the chain B homodimer of TTM101-LC02 on MDA-MB-231 (GPC3-negative cells, non-target cells) is only 16.2% (reduced by approximately 75.1% compared to IFN-α 2b, and reduced by approximately 50% compared to IFN-α 2b-Fc). In summary, the potentially risky impurities have only an extremely weak effect on non-target cells, i.e., the potential safety risks or potential toxic side effects are extremely low.

### Example 9. Detection of in vivo anti-tumor efficacy of bispecific recombinant protein containing IFN-α 2b low-affinity mutant

### Anti-tumor efficacy of GPC3-targeted bispecific recombinant protein on HuH-7 subcutaneous tumor model in BALB/c nude mice

BALB/c nude mice were inoculated with a HuH-7 tumor mass of approximately 30 mm³ on the right nape, and the experimental animals were ear tagged at the same time as the only confirmatory marker for subsequent experiments. Waiting for tumor growth, when the average tumor volume reached 150 mm³, the mice were randomized into groups and administrated, with 6 mice in each group. All mice were administered intravenously in a volume of 10 mL/kg for 3 weeks, twice a week for a total of 6 doses.

The experimental index is to investigate whether the tumor growth is inhibited, delayed, or cured. The tumor diameter was measured twice a week using a vernier caliper. The calculation formula for tumor volume is: V = 0.5a × b², where a and b represent the long diameter and short diameter of the tumor, respectively. Statistical analysis included the mean and standard error (SEM) of the tumor volume at each time point for each group. The comparison between the two groups was analyzed by one-tailed T test, and GraphPad Prism was used for all data analysis. p<0.05 is considered to be a significant difference.

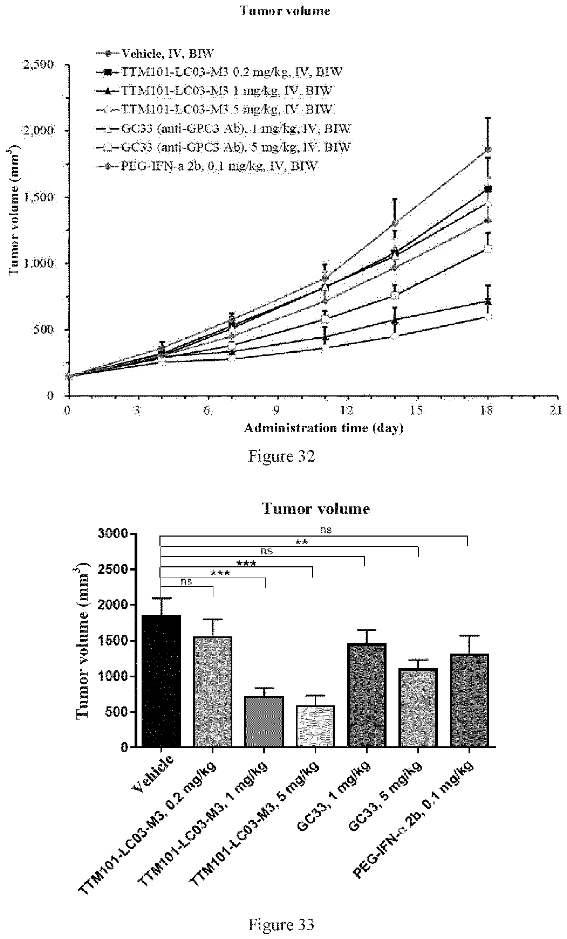

As shown in Figure 32 and Figure 33, TTM101-LC03-M3 inhibited tumor growth in a dose-dependent manner, and its anti-tumor efficacy was better than that of GC33 at the same dose and also better than that of PEG-IFN-α 2b at a high dose (higher than the tolerated dose in clinical trials). The above results show that GPC3-targeted IFN-α 2b with A145G attenuating mutation can effectively inhibit tumor growth.

### Anti-tumor efficacy of PD-L1-targeted bispecific recombinant protein in the MDA-MB-231 model of PBMC humanized M-NSG mice

M-NSG mice (6 to 8 weeks, female) were subcutaneously inoculated with MDA-MB-231 cells (number of inoculated cells: 1E7/mouse + 30% Matrigel), which was recorded as day 0. The survival status of the mice and subcutaneous tumor formation were observed the next day. 7 days after inoculation, PBMC cells (0205C) were injected into the tail vein (number of inoculated cells: 5E6/mouse). 7 days after PBMC inoculation, the whole blood of mice was collected to detect hCD45% by flow cytometry. Based on the average tumor volume (approximately 200 mm³) and hCD45% ratio, the mice were randomized into groups, and the day of grouping was considered as D0. All mice were administered intravenously in a volume of 10 mL/kg for 3 weeks, twice a week for a total of 6 doses.

The experimental index is to investigate whether the tumor growth is inhibited, delayed, or cured. The tumor diameter was measured twice a week using a vernier caliper. The calculation formula for tumor volume is: V = 0.5a × b², where a and b represent the long diameter and short diameter of the tumor, respectively. Statistical analysis included the mean and standard error (SEM) of the tumor volume at each time point for each group. The comparison between the two groups was analyzed by one-tailed T test, and GraphPad Prism was used for all data analysis. p<0.05 is considered to be a significant difference.

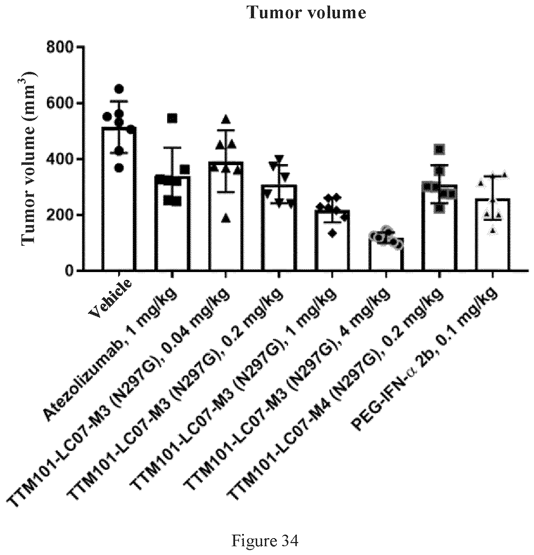

As shown in Figure 34, TTM101-LC07-M3 (N297G) inhibited tumor growth in a dose-dependent manner, and its anti-tumor efficacy was better than that of Atezolizumab at the same dose. The efficacy of 4 mg/kg TTM101-LC07-M3 (N297G) was better than that of PEG-IFN-α 2b at a high dose (higher than the tolerated dose in clinical trials). At a dose level of 0.2 mg/kg, TTM101-LC07-M4 (N297G) was comparable in efficacy to 1 mg/kg Atezolizumab. The above results show that PD-L1-targeted IFN-α 2b with A145G and R149A attenuating mutations can effectively inhibit tumor growth.

### Example 10. Investigation of freeze-thaw stability of bispecific recombinant protein

The existing recombinant human albumin interferon-α 2b fusion protein has poor freeze-thaw stability and is not suitable for repeated freezing and thawing. For example, PEGylated long-acting interferon cannot be frozen and shaken, and requires high transportation and storage conditions. In order to study the freeze-thaw stability of the bispecific recombinant protein of the present disclosure, repeated freeze-thaw stability tests were performed on the bispecific recombinant protein having the first type of structure. The protein was placed in 20 mM NaAc (pH = 5) buffer and subjected to 5 repeated freeze-thaw cycles at -40°C. Samples before and after freeze-thaw were analyzed for purity (size exclusion chromatography, SEC) and appearance. As shown in Table7, TTM101-LC03 and mutants thereof have good freeze-thaw stability, with purity above 95%, and the appearance is clear after 5 repeated freeze-thaw cycles, indicating that the freeze-thaw stability of the IFN-α 2b protein in the bispecific recombinant protein of the present disclosure is significantly better than that of IFN-α 2b monomer or PEGylated IFN-α 2b.

**Table 7: Freeze-thaw stability of bispecific recombinant proteins having the first type of structure**

| No. | SEC-HPLC (%) | Unfreeze-thawed | | | 5 freeze-thaw cycles | | |
|---|---|---|---|---|---|---|---|
| | | Monomer | Polymer | Impurities with low molecular weight | Monomer | Polymer | Impurities with low molecular weight |
| 1 | TTM101-LC03 | 96.50 | 3.50 | ND | 97.87 | 2.14 | ND |
| 2 | TTM101-LC03-M3 | 99.11 | 0.89 | ND | 97.68 | 2.32 | ND |
| 3 | TTM101-LC03-M4 | 98.03 | 1.97 | ND | 95.74 | 4.13 | 0.12 |

The bispecific recombinant protein of the present disclosure whose first functional binding region targets other targets (e.g., 5T4, AGS-16, ALK1, ANG-2, B7-H3, B7-H4, c-fms, c-Met, CA6, CD123, CD19, CD20, CD22, CD24, EpCAM, CD30, CD32b, CD37, CD38, CD40, CD52, CD70, CD74, CD79b, CD98, CEA, CEACAM5, CLDN18.2, CLDN6, CS1, CXCR4, DLL-4, EGFR, EGFRvIII, EGP-1, ENPP3, EphA3, ETBR, FGFR2, FN, FR-α, GCC, GD2, GPNMB, HER2, HER3, HLA-DR, ICAM-1, IGF-1R, IL-3R, LIV-1, MSLN, MUC16, MUC1, NaPi2b, Nectin-4, Notch 2, Notch 1, PD-L2, PDGFR-α, PS, PSMA, SLTRK6, STEAP1, TEM1, VEGFR, CD25, CD27L, DKK-1, CSF-1R, MSB0010718C, CD138, Sig1ec15, and CD155) can also achieve similar effects as the above targets.

The use of any and all examples, or exemplary language (e.g., "for example") provided herein, is intended merely to better illustrate the present disclosure, and does not pose a limitation on the scope of the present disclosure, unless otherwise claimed. No language in the specification should be construed as indicating any non-claimed element as essential to the practice of the present disclosure.

All publications and patent applications cited in this specification are incorporated herein by reference in their entireties to the same extent as if each individual publication or patent application is specifically and individually indicated to be incorporated by reference. Furthermore, any theory, mechanism, proof, or finding stated herein is meant to further enhance the understanding of the present disclosure, and is not intended to limit the present disclosure in any way to such theory, mechanism, proof, or finding. Although the present disclosure has been illustrated and described in detail in the drawings and foregoing description, such illustration and description are to be considered illustrative and not restrictive.

## Claims

1. A bispecific recombinant protein, wherein the bispecific recombinant protein includes a first functional binding region, a second functional binding region, and an Fc region; the first functional binding region includes a first chain and a second chain, and C-terminus of the first chain and C-terminus of the second functional binding region are separately linked to N-terminus of the Fc region directly or via a linker sequence; C-terminus of the second chain is linked to N-terminus of the second functional binding region directly or via a linker sequence;
the first functional binding region includes a heavy chain variable region (VH), a light chain variable region (VL), a heavy chain constant region 1 (CH1), and a light chain constant region (CL);
the second functional binding region comprises an interferon, a truncated variant thereof, or a mutant thereof;
preferably, C-terminus of the CL or CH1 is linked to N-terminus of the Fc region directly or via a linker sequence; C-terminus of the corresponding CH1 or CL is linked to N-terminus of the second functional binding region directly or via a linker sequence.

2. The bispecific recombinant protein according to claim 1, wherein the interferon is type I interferon; preferably, the type I interferon is IFNα; more preferably, the IFNα is selected from one or more of IFN-α1a, IFN-α1b, IFN-α2a, IFN-α2b, IFN-α4a, IFN-α4b, IFN-α5, IFN-α6, IFN-α7, IFN-α8, IFN-α10, IFN-α14, IFN-α16, IFN-α17, and IFN-α21.

3. The bispecific recombinant protein according to claim 2, wherein the interferon is IFN-α2b;
preferably, the second functional binding region comprises an amino acid sequence selected from any one of the following a1) and a2): a1) SEQ ID NO: 17; a2) an amino acid sequence obtained by addition, deletion, modification, and/or substitution of at least one amino acid residue of the amino acid sequence as shown in SEQ ID NO: 17, whose monomer has binding affinity to IFNα receptor and the binding affinity is no higher than that of an a1) monomer to IFNα receptor.

4. The bispecific recombinant protein according to claim 3, wherein amino acid sequence of a2) monomer is the amino acid sequence obtained by substitution at a corresponding site of the amino acid sequence as shown in SEQ ID NO: 17; the substitution at the corresponding site is selected from one or more of L26A, L30A, A145G, R149A, and S152A;
preferably, the a2) monomer has a single-site substitution of L26A, L30A, A145G, R149A, or S152A, and the corresponding amino acid sequences are shown in SEQ ID NOs: 18 to 22, respectively.

5. The bispecific recombinant protein according to claim 1, wherein the VH is linked to CH1 or CL directly or via a linker sequence; the VL is linked to CL or CH1 directly or via a linker sequence;
preferably, when VL is linked to CL, VH is linked to CH1; when VL is linked to CH1, VH is linked to CL.

6. The bispecific recombinant protein according to claim 1, wherein the first functional binding region targets a tumor cell or an immune cell; preferably, the first functional binding region targets any one or more of the following targets: GPC3, BCMA, PD-L1, Trop-2, 5T4, AGS-16, ALK1, ANG-2, B7-H3, B7-H4, c-fms, c-Met, CA6, CD123, CD19, CD20, CD22, CD24, EpCAM, CD30, CD32b, CD37, CD38, CD40, CD52, CD70, CD74, CD79b, CD98, CEA, CEACAM5, CLDN18.2, CLDN6, CS1, CXCR4, DLL-4, EGFR, EGFRvIII, EGP-1, ENPP3, EphA3, ETBR, FGFR2, FN, FR-α, GCC, GD2, GPNMB, HER2, HER3, HLA-DR, ICAM-1, IGF-1R, IL-3R, LIV-1, MSLN, MUC16, MUC1, NaPi2b, Nectin-4, Notch 2, Notch 1, PD-L2, PDGFR-α, PS, PSMA, SLTRK6, STEAP1, TEM1, VEGFR, CD25, CD27L, DKK-1, CSF-1R, MSB0010718C, CD138, Sig1ec15, and CD155.

7. The bispecific recombinant protein according to claim 6, wherein the first functional binding region targets GPC3; the first functional binding region comprises an antigen-binding fragment of a specific anti-GPC3 monoclonal antibody; preferably, heavy chain variable region (VH) sequence of the specific anti-GPC3 monoclonal antibody is shown in SEQ ID NO: 1 and light chain variable region (VL) sequence is shown in SEQ ID NO: 2;
or, the first functional binding region targets PD-L1; the first functional binding region comprises an antigen-binding fragment of a specific anti-PD-L1 monoclonal antibody; preferably, heavy chain variable region (VH) sequence of the specific anti-PD-L1 monoclonal antibody is shown in SEQ ID NO: 5 and light chain variable region (VL) sequence is shown in SEQ ID NO: 6;
or, the first functional binding region targets BCMA; the first functional binding region comprises an antigen-binding fragment of a specific anti-BCMA monoclonal antibody; preferably, heavy chain variable region (VH) sequence of the specific anti-BCMA monoclonal antibody is shown in SEQ ID NO: 7 or SEQ ID NO: 9 and corresponding light chain variable region (VL) sequence is shown in SEQ ID NO: 8 or SEQ ID NO: 10;
or, the first functional binding region targets Trop-2; the first functional binding region comprises an antigen-binding fragment of a specific anti-Trop-2 monoclonal antibody; preferably, heavy chain variable region (VH) sequence of the specific anti-Trop-2 monoclonal antibody is shown in SEQ ID NO: 11 and light chain variable region (VL) sequence is shown in SEQ ID NO: 12.

8. The bispecific recombinant protein according to any one of claims 1 to 7, wherein the linker sequence has a length of no more than 20 amino acid residues, and the linker sequence is selected from the group consisting of (GGGGS)n, (GGGS)n, (GGS)n, (G)n, (GS)n, (EAAAK)n, and (XP)n, wherein n is a natural number;
preferably, amino acid sequence of the linker sequence is shown in SEQ ID NOs: 13 to 16.

9. The bispecific recombinant protein according to claim 8, wherein the bispecific recombinant protein consists of a chain A and a chain B; wherein structure of the chain A is VH-CH1-FcAregion, and structure of the chain B is VL-CL-second functional binding region-FcB region; or, structure of the chain A is VH-CL-FcA region, and structure of the chain B is VL-CH1-second functional binding region-FcB region; or, structure of the chain A is VL-CH1-FcA region, and structure of the chain B is VH-CL-second functional binding region-FcB region; or, structure of the chain A is VL-CL-FcA region, and structure of the chain B is VH-CH1-second functional binding region-FcB region; the chain A and the chain B are combined via one or more bindings selected from intermolecular forces, covalent bonds, and salt bonds.

10. A bispecific recombinant protein, wherein the bispecific recombinant protein includes a first functional binding region, a second functional binding region, and an Fc region; the first functional binding region includes a heavy chain variable region (VH), a light chain variable region (VL), a heavy chain constant region 1 (CH1), and a light chain constant region (CL); the second functional binding region comprises an interferon, a truncated variant thereof, or a mutant thereof; the bispecific recombinant protein consists of a first arm and a second arm, and the first arm consists of a chain 1 and a chain 2; wherein structure of the chain 1 is VH-CH1-FcA region, and structure of the chain 2 is VL-CL; or, structure of the chain 1 is VH-CL-FcA region, and structure of the chain 2 is VL-CH1; or, structure of the chain 1 is VL-CH1-FcA region, and structure of the chain 2 is VH-CL; or, structure of the chain 1 is VL-CL-FcA region, and structure of the chain 2 is VH-CH1; structure of the second arm is second functional binding region-FcB region, and the FcA region and the FcB region constitute the Fc region of the bispecific recombinant protein; in the second arm, amino acid sequence of the second functional binding region comprises any one of SEQ ID NOs: 17 to 22;
when the first functional binding region targets GPC3, the first functional binding region comprises an antigen-binding fragment of a specific anti-GPC3 monoclonal antibody; preferably, in the first arm, amino acid sequence of VH comprises SEQ ID NO: 1, amino acid sequence of CH1 comprises SEQ ID NO: 31, amino acid sequence of VL comprises SEQ ID NO: 2, and amino acid sequence of CL comprises SEQ ID NO: 32;
when the first functional binding region targets PD-L1, the first functional binding region comprises an antigen-binding fragment of a specific anti-PD-L1 monoclonal antibody; preferably, in the first arm, amino acid sequence of VH comprises SEQ ID NO: 5, amino acid sequence of CH1 comprises SEQ ID NO: 31, amino acid sequence of VL comprises SEQ ID NO: 6, and amino acid sequence of CL comprises SEQ ID NO: 32;
when the first functional binding region targets BCMA, the first functional binding region comprises an antigen-binding fragment of a specific anti-BCMA monoclonal antibody; preferably, in the first arm, amino acid sequence of VH comprises SEQ ID NO: 7 or 9, amino acid sequence of CH1 comprises SEQ ID NO: 31, amino acid sequence of VL comprises SEQ ID NO: 8 or 10, and amino acid sequence of CL comprises SEQ ID NO: 32;
when the first functional binding region targets Trop-2, the first functional binding region comprises an antigen-binding fragment of a specific anti-Trop-2 monoclonal antibody; preferably, in the first arm, amino acid sequence of VH comprises SEQ ID NO: 11, amino acid sequence of CH1 comprises SEQ ID NO: 31, amino acid sequence of VL comprises SEQ ID NO: 12, and amino acid sequence of CL comprises SEQ ID NO: 32.

11. The bispecific recombinant protein according to claim 9 or 10, wherein the Fc region of the bispecific recombinant protein is Fc region derived from IgG1, IgG2, IgG3, or IgG4; the Fc region comprises a natural sequence or a non-natural sequence;
preferably, the Fc region is Fc region derived from human IgG1, IgG2, IgG3, or IgG4;
more preferably, the FcA region and the FcB region are heterodimerized through knobs-into-holes;
further more preferably, when amino acid sequence of the FcA region comprises SEQ ID NO: 25, amino acid sequence of the FcB region comprises SEQ ID NO: 25; or, when amino acid sequence of the FcA region comprises SEQ ID NO: 26, amino acid sequence of the FcB region comprises SEQ ID NO: 27; when amino acid sequence of the FcA region comprises SEQ ID NO: 27, amino acid sequence of the FcB region comprises SEQ ID NO: 26; or, when amino acid sequence of the FcA region comprises SEQ ID NO: 28, amino acid sequence of the FcB region comprises SEQ ID NO: 28; or, when amino acid sequence of the FcA region comprises SEQ ID NO: 29, amino acid sequence of the FcB region comprises SEQ ID NO: 30; or, when amino acid sequence of the FcA region comprises SEQ ID NO: 30, amino acid sequence of the FcB region comprises SEQ ID NO: 29.

12. The bispecific recombinant protein according to claim 11, wherein the first functional binding region targets GPC3; in the chain A, amino acid sequence of VH comprises SEQ ID NO: 1, amino acid sequence of VL comprises SEQ ID NO: 2, amino acid sequence of CH1 comprises SEQ ID NO: 31, amino acid sequence of CL comprises SEQ ID NO: 32, and amino acid sequence of the FcA region comprises SEQ ID NO: 26 or SEQ ID NO: 27;
in the chain B, amino acid sequence of VH comprises SEQ ID NO: 1, amino acid sequence of VL comprises SEQ ID NO: 2, amino acid sequence of CH1 comprises SEQ ID NO: 31, amino acid sequence of CL comprises SEQ ID NO: 32, amino acid sequence of the second functional binding region comprises SEQ ID NO: 17, 18, 19, 21, or 22, and amino acid sequence of the FcB region comprises SEQ ID NO: 27 or SEQ ID NO: 26;
preferably, among the amino acid sequences of the chain B, C-terminus of SEQ ID NO: 2 or SEQ ID NO: 1 is linked to N-terminus of SEQ ID NO: 32 directly, C-terminus of SEQ ID NO: 32 is linked to N-terminus of SEQ ID NO: 17, SEQ ID NO: 18, SEQ ID NO: 19, SEQ ID NO: 21, or SEQ ID NO: 22 via a linker sequence, and the linker sequence is selected from any one of SEQ ID NOs: 13 to 16;
more preferably, when amino acid sequence of the chain A consists of sequentially linked SEQ ID NO: 1, 31, and the FcA region,
amino acid sequence of the chain B consists of sequentially linked SEQ ID NO: 2, 32, 13, 17, and the FcB region;
or, amino acid sequence of the chain B consists of sequentially linked SEQ ID NO: 2, 32, 13, 18, and the FcB region;
or, amino acid sequence of the chain B consists of sequentially linked SEQ ID NO: 2, 32, 13, 19, and the FcB region;
or, amino acid sequence of the chain B consists of sequentially linked SEQ ID NO: 2, 32, 13, 20, and the FcB region;
or, amino acid sequence of the chain B consists of sequentially linked SEQ ID NO: 2, 32, 13, 21, and the FcB region;
or, amino acid sequence of the chain B consists of sequentially linked SEQ ID NO: 2, 32, 13, 22, and the FcB region;
or, amino acid sequence of the chain B consists of sequentially linked SEQ ID NO: 2, 32, 14, 17, and the FcB region;
or, amino acid sequence of the chain B consists of sequentially linked SEQ ID NO: 2, 32, 14, 18, and the FcB region;
or, amino acid sequence of the chain B consists of sequentially linked SEQ ID NO: 2, 32, 14, 19, and the FcB region;
or, amino acid sequence of the chain B consists of sequentially linked SEQ ID NO: 2, 32, 14, 20, and the FcB region;
or, amino acid sequence of the chain B consists of sequentially linked SEQ ID NO: 2, 32, 14, 21, and the FcB region;
or, amino acid sequence of the chain B consists of sequentially linked SEQ ID NO: 2, 32, 14, 22, and the FcB region;
or, amino acid sequence of the chain B consists of sequentially linked SEQ ID NO: 2, 32, 15, 17, and the FcB region;
or, amino acid sequence of the chain B consists of sequentially linked SEQ ID NO: 2, 32, 15, 18, and the FcB region;
or, amino acid sequence of the chain B consists of sequentially linked SEQ ID NO: 2, 32, 15, 19, and the FcB region;
or, amino acid sequence of the chain B consists of sequentially linked SEQ ID NO: 2, 32, 15, 20, and the FcB region;
or, amino acid sequence of the chain B consists of sequentially linked SEQ ID NO: 2, 32, 15, 21, and the FcB region;
or, amino acid sequence of the chain B consists of sequentially linked SEQ ID NO: 2, 32, 15, 22, and the FcB region;
or, amino acid sequence of the chain B consists of sequentially linked SEQ ID NO: 2, 32, 16, 17, and the FcB region;
or, amino acid sequence of the chain B consists of sequentially linked SEQ ID NO: 2, 32, 16, 18, and the FcB region;
or, amino acid sequence of the chain B consists of sequentially linked SEQ ID NO: 2, 32, 16, 19, and the FcB region;
or, amino acid sequence of the chain B consists of sequentially linked SEQ ID NO: 2, 32, 16, 21, and the FcB region;
or, amino acid sequence of the chain B consists of sequentially linked SEQ ID NO: 2, 32, 16, 22, and the FcB region;
when amino acid sequence of the FcA region is SEQ ID NO: 26, amino acid sequence of the FcB region is SEQ ID NO: 27; or, when amino acid sequence of the FcA region is SEQ ID NO: 27, amino acid sequence of the FcB region is SEQ ID NO: 26.

13. The bispecific recombinant protein according to claim 11, wherein the first functional binding region targets PD-L1; in the chain A, amino acid sequence of VH comprises SEQ ID NO: 5, amino acid sequence of VL comprises SEQ ID NO: 6, amino acid sequence of CH1 comprises SEQ ID NO: 31, amino acid sequence of CL comprises SEQ ID NO: 32, and amino acid sequence of the FcA region comprises SEQ ID NO: 26, SEQ ID NO: 27, SEQ ID NO: 29, or SEQ ID NO: 30;
in the chain B, amino acid sequence of VH comprises SEQ ID NO: 5, amino acid sequence of VL comprises SEQ ID NO: 6, amino acid sequence of CH1 comprises SEQ ID NO: 31, amino acid sequence of CL comprises SEQ ID NO: 32, amino acid sequence of the second functional binding region comprises any one of SEQ ID NOs: 17 to 22, and amino acid sequence of the FcB region comprises SEQ ID NO: 27, SEQ ID NO: 26, SEQ ID NO: 30, or SEQ ID NO: 29;
preferably, among the amino acid sequences of the chain B, C-terminus of SEQ ID NO: 6 or SEQ ID NO: 5 is linked to N-terminus of SEQ ID NO: 32 directly, C-terminus of SEQ ID NO: 32 is linked to N-terminus of any one of SEQ ID NOs: 17 to 22 via a linker sequence, and the linker sequence is selected from any one of SEQ ID NOs: 13 to 16;
more preferably,
when amino acid sequence of the chain A consists of sequentially linked SEQ ID NO: 5, 31, and the FcA region,
amino acid sequence of the chain B consists of sequentially linked SEQ ID NO: 6, 32, 13, 17, and the FcB region;
or, amino acid sequence of the chain B consists of sequentially linked SEQ ID NO: 6, 32, 13, 18, and the FcB region;
or, amino acid sequence of the chain B consists of sequentially linked SEQ ID NO: 6, 32, 13, 19, and the FcB region;
or, amino acid sequence of the chain B consists of sequentially linked SEQ ID NO: 6, 32, 13, 20, and the FcB region;
or, amino acid sequence of the chain B consists of sequentially linked SEQ ID NO: 6, 32, 13, 21, and the FcB region;
or, amino acid sequence of the chain B consists of sequentially linked SEQ ID NO: 6, 32, 13, 22, and the FcB region;
or, amino acid sequence of the chain B consists of sequentially linked SEQ ID NO: 6, 32, 14, 17, and the FcB region;
or, amino acid sequence of the chain B consists of sequentially linked SEQ ID NO: 6, 32, 14, 18, and the FcB region;
or, amino acid sequence of the chain B consists of sequentially linked SEQ ID NO: 6, 32, 14, 19, and the FcB region;
or, amino acid sequence of the chain B consists of sequentially linked SEQ ID NO: 6, 32, 14, 20, and the FcB region;
or, amino acid sequence of the chain B consists of sequentially linked SEQ ID NO: 6, 32, 14, 21, and the FcB region;
or, amino acid sequence of the chain B consists of sequentially linked SEQ ID NO: 6, 32, 14, 22, and the FcB region;
or, amino acid sequence of the chain B consists of sequentially linked SEQ ID NO: 6, 32, 15, 17, and the FcB region;
or, amino acid sequence of the chain B consists of sequentially linked SEQ ID NO: 6, 32, 15, 18, and the FcB region;
or, amino acid sequence of the chain B consists of sequentially linked SEQ ID NO: 6, 32, 15, 19, and the FcB region;
or, amino acid sequence of the chain B consists of sequentially linked SEQ ID NO: 6, 32, 15, 20, and the FcB region;
or, amino acid sequence of the chain B consists of sequentially linked SEQ ID NO: 6, 32, 15, 21, and the FcB region;
or, amino acid sequence of the chain B consists of sequentially linked SEQ ID NO: 6, 32, 15, 22, and the FcB region;
or, amino acid sequence of the chain B consists of sequentially linked SEQ ID NO: 6, 32, 16, 17, and the FcB region;
or, amino acid sequence of the chain B consists of sequentially linked SEQ ID NO: 6, 32, 16, 18, and the FcB region;
or, amino acid sequence of the chain B consists of sequentially linked SEQ ID NO: 6, 32, 16, 19, and the FcB region;
or, amino acid sequence of the chain B consists of sequentially linked SEQ ID NO: 6, 32, 16, 20, and the FcB region;
or, amino acid sequence of the chain B consists of sequentially linked SEQ ID NO: 6, 32, 16, 21, and the FcB region;
or, amino acid sequence of the chain B consists of sequentially linked SEQ ID NO: 6, 32, 16, 22, and the FcB region;
when amino acid sequence of the FcA region is SEQ ID NO: 26, amino acid sequence of the FcB region is SEQ ID NO: 27; or, when amino acid sequence of the FcA region is SEQ ID NO: 27, amino acid sequence of the FcB region is SEQ ID NO: 26; or, when amino acid sequence of the FcA region is SEQ ID NO: 29, amino acid sequence of the FcB region is SEQ ID NO: 30; or, when amino acid sequence of the FcA region is SEQ ID NO: 30, amino acid sequence of the FcB region is SEQ ID NO: 29.

14. The bispecific recombinant protein according to claim 11, wherein the first functional binding region targets BCMA;
preferably, in the chain A, amino acid sequence of VH comprises SEQ ID NO: 7 or 9, amino acid sequence of VL comprises SEQ ID NO: 8 or 10, amino acid sequence of CH1 comprises SEQ ID NO: 31, amino acid sequence of CL comprises SEQ ID NO: 32, and amino acid sequence of the FcA region comprises SEQ ID NO: 26 or SEQ ID NO: 27;
in the chain B, amino acid sequence of VH comprises SEQ ID NO: 7 or 9, amino acid sequence of VL comprises SEQ ID NO: 8 or 10, amino acid sequence of CH1 comprises SEQ ID NO: 31, amino acid sequence of CL comprises SEQ ID NO: 32, amino acid sequence of the second functional binding region comprises any one of SEQ ID NOs: 17 to 22, and amino acid sequence of the FcB region comprises SEQ ID NO: 27 or SEQ ID NO: 26;
more preferably, among the amino acid sequences of the chain B, C-terminus of SEQ ID NO: 7, 8, 9, or 10 is linked to N-terminus of SEQ ID NO: 32 directly, C-terminus of SEQ ID NO: 32 is linked to N-terminus of any one of SEQ ID NOs: 17 to 22 via a linker sequence, and the linker sequence is selected from any one of SEQ ID NOs: 13 to 16;
further more preferably,
when amino acid sequence of the chain A consists of sequentially linked SEQ ID NO: 7, 31, and the FcA region,
amino acid sequence of the chain B consists of sequentially linked SEQ ID NO: 8, 32, 13, 17, and the FcB region;
or, amino acid sequence of the chain B consists of sequentially linked SEQ ID NO: 8, 32, 13, 18, and the FcB region;
or, amino acid sequence of the chain B consists of sequentially linked SEQ ID NO: 8, 32, 13, 19, and the FcB region;
or, amino acid sequence of the chain B consists of sequentially linked SEQ ID NO: 8, 32, 13, 20, and the FcB region;
or, amino acid sequence of the chain B consists of sequentially linked SEQ ID NO: 8, 32, 13, 21, and the FcB region;
or, amino acid sequence of the chain B consists of sequentially linked SEQ ID NO: 8, 32, 13, 22, and the FcB region;
or, amino acid sequence of the chain B consists of sequentially linked SEQ ID NO: 8, 32, 14, 17, and the FcB region;
or, amino acid sequence of the chain B consists of sequentially linked SEQ ID NO: 8, 32, 14, 18, and the FcB region;
or, amino acid sequence of the chain B consists of sequentially linked SEQ ID NO: 8, 32, 14, 19, and the FcB region;
or, amino acid sequence of the chain B consists of sequentially linked SEQ ID NO: 8, 32, 14, 20, and the FcB region;
or, amino acid sequence of the chain B consists of sequentially linked SEQ ID NO: 8, 32, 14, 21, and the FcB region;
or, amino acid sequence of the chain B consists of sequentially linked SEQ ID NO: 8, 32, 14, 22, and the FcB region;
or, amino acid sequence of the chain B consists of sequentially linked SEQ ID NO: 8, 32, 15, 17, and the FcB region;
or, amino acid sequence of the chain B consists of sequentially linked SEQ ID NO: 8, 32, 15, 18, and the FcB region;
or, amino acid sequence of the chain B consists of sequentially linked SEQ ID NO: 8, 32, 15, 19, and the FcB region;
or, amino acid sequence of the chain B consists of sequentially linked SEQ ID NO: 8, 32, 15, 20, and the FcB region;
or, amino acid sequence of the chain B consists of sequentially linked SEQ ID NO: 8, 32, 15, 21, and the FcB region;
or, amino acid sequence of the chain B consists of sequentially linked SEQ ID NO: 8, 32, 15, 22, and the FcB region;
or, amino acid sequence of the chain B consists of sequentially linked SEQ ID NO: 8, 32, 16, 17, and the FcB region;
or, amino acid sequence of the chain B consists of sequentially linked SEQ ID NO: 8, 32, 16, 18, and the FcB region;
or, amino acid sequence of the chain B consists of sequentially linked SEQ ID NO: 8, 32, 16, 19, and the FcB region;
or, amino acid sequence of the chain B consists of sequentially linked SEQ ID NO: 8, 32, 16, 20, and the FcB region;
or, amino acid sequence of the chain B consists of sequentially linked SEQ ID NO: 8, 32, 16, 21, and the FcB region;
or, amino acid sequence of the chain B consists of sequentially linked SEQ ID NO: 8, 32, 16, 22, and the FcB region;
when amino acid sequence of the chain A consists of sequentially linked SEQ ID NO: 9, 31, and the FcA region,
amino acid sequence of the chain B consists of sequentially linked SEQ ID NO: 10, 32, 13, 17, and the FcB region;
or, amino acid sequence of the chain B consists of sequentially linked SEQ ID NO: 10, 32, 13, 18, and the FcB region;
or, amino acid sequence of the chain B consists of sequentially linked SEQ ID NO: 10, 32, 13, 19, and the FcB region;
or, amino acid sequence of the chain B consists of sequentially linked SEQ ID NO: 10, 32, 13, 20, and the FcB region;
or, amino acid sequence of the chain B consists of sequentially linked SEQ ID NO: 10, 32, 13, 21, and the FcB region;
or, amino acid sequence of the chain B consists of sequentially linked SEQ ID NO: 10, 32, 13, 22, and the FcB region;
or, amino acid sequence of the chain B consists of sequentially linked SEQ ID NO: 10, 32, 14, 17, and the FcB region;
or, amino acid sequence of the chain B consists of sequentially linked SEQ ID NO: 10, 32, 14, 18, and the FcB region;
or, amino acid sequence of the chain B consists of sequentially linked SEQ ID NO: 10, 32, 14, 19, and the FcB region;
or, amino acid sequence of the chain B consists of sequentially linked SEQ ID NO: 10, 32, 14, 20, and the FcB region;
or, amino acid sequence of the chain B consists of sequentially linked SEQ ID NO: 10, 32, 14, 21, and the FcB region;
or, amino acid sequence of the chain B consists of sequentially linked SEQ ID NO: 10, 32, 14, 22, and the FcB region;
or, amino acid sequence of the chain B consists of sequentially linked SEQ ID NO: 10, 32, 15, 17, and the FcB region;
or, amino acid sequence of the chain B consists of sequentially linked SEQ ID NO: 10, 32, 15, 18, and the FcB region;
or, amino acid sequence of the chain B consists of sequentially linked SEQ ID NO: 10, 32, 15, 19, and the FcB region;
or, amino acid sequence of the chain B consists of sequentially linked SEQ ID NO: 10, 32, 15, 20, and the FcB region;
or, amino acid sequence of the chain B consists of sequentially linked SEQ ID NO: 10, 32, 15, 21, and the FcB region;
or, amino acid sequence of the chain B consists of sequentially linked SEQ ID NO: 10, 32, 15, 22, and the FcB region;
or, amino acid sequence of the chain B consists of sequentially linked SEQ ID NO: 10, 32, 16, 17, and the FcB region;
or, amino acid sequence of the chain B consists of sequentially linked SEQ ID NO: 10, 32, 16, 18, and the FcB region;
or, amino acid sequence of the chain B consists of sequentially linked SEQ ID NO: 10, 32, 16, 19, and the FcB region;
or, amino acid sequence of the chain B consists of sequentially linked SEQ ID NO: 10, 32, 16, 20, and the FcB region;
or, amino acid sequence of the chain B consists of sequentially linked SEQ ID NO: 10, 32, 16, 21, and the FcB region;
or, amino acid sequence of the chain B consists of sequentially linked SEQ ID NO: 10, 32, 16, 22, and the FcB region;
when amino acid sequence of the FcA region is SEQ ID NO: 26, amino acid sequence of the FcB region is SEQ ID NO: 27; when amino acid sequence of the FcA region is SEQ ID NO: 27, amino acid sequence of the FcB region is SEQ ID NO: 26.

15. The bispecific recombinant protein according to claim 11, wherein the first functional binding region targets Trop-2;
in the chain A, amino acid sequence of VH comprises SEQ ID NO: 11, amino acid sequence of VL comprises SEQ ID NO: 12, amino acid sequence of CH1 comprises SEQ ID NO: 31, amino acid sequence of CL comprises SEQ ID NO: 32, and amino acid sequence of the FcA region comprises SEQ ID NO: 26 or SEQ ID NO: 27;
in the chain B, amino acid sequence of VH comprises SEQ ID NO: 11, amino acid sequence of VL comprises SEQ ID NO: 12, amino acid sequence of CH1 comprises SEQ ID NO: 31, amino acid sequence of CL comprises SEQ ID NO: 32, amino acid sequence of the second functional binding region comprises any one of SEQ ID NOs: 17 to 22, and amino acid sequence of the FcB region comprises SEQ ID NO: 27 or SEQ ID NO: 26;
preferably, among the amino acid sequences of the chain B, C-terminus of SEQ ID NO: 12 or SEQ ID NO: 11 is linked to N-terminus of SEQ ID NO: 32 directly, C-terminus of SEQ ID NO: 32 is linked to N-terminus of any one of SEQ ID NOs: 17 to 22 via a linker sequence, and the linker sequence is selected from any one of SEQ ID NOs: 13 to 16;
more preferably,
when amino acid sequence of the chain A consists of sequentially linked SEQ ID NO: 11, 31, and the FcA region,
amino acid sequence of the chain B consists of sequentially linked SEQ ID NO: 12, 32, 13, 17, and the FcB region;
or, amino acid sequence of the chain B consists of sequentially linked SEQ ID NO: 12, 32, 13, 18, and the FcB region;
or, amino acid sequence of the chain B consists of sequentially linked SEQ ID NO: 12, 32, 13, 19, and the FcB region;
or, amino acid sequence of the chain B consists of sequentially linked SEQ ID NO: 12, 32, 13, 20, and the FcB region;
or, amino acid sequence of the chain B consists of sequentially linked SEQ ID NO: 12, 32, 13, 21, and the FcB region;
or, amino acid sequence of the chain B consists of sequentially linked SEQ ID NO: 12, 32, 13, 22, and the FcB region;
or, amino acid sequence of the chain B consists of sequentially linked SEQ ID NO: 12, 32, 14, 17, and the FcB region;
or, amino acid sequence of the chain B consists of sequentially linked SEQ ID NO: 12, 32, 14, 18, and the FcB region;
or, amino acid sequence of the chain B consists of sequentially linked SEQ ID NO: 12, 32, 14, 19, and the FcB region;
or, amino acid sequence of the chain B consists of sequentially linked SEQ ID NO: 12, 32, 14, 20, and the FcB region;
or, amino acid sequence of the chain B consists of sequentially linked SEQ ID NO: 12, 32, 14, 21, and the FcB region;
or, amino acid sequence of the chain B consists of sequentially linked SEQ ID NO: 12, 32, 14, 22, and the FcB region;
or, amino acid sequence of the chain B consists of sequentially linked SEQ ID NO: 12, 32, 15, 17, and the FcB region;
or, amino acid sequence of the chain B consists of sequentially linked SEQ ID NO: 12, 32, 15, 18, and the FcB region;
or, amino acid sequence of the chain B consists of sequentially linked SEQ ID NO: 12, 32, 15, 19, and the FcB region;
or, amino acid sequence of the chain B consists of sequentially linked SEQ ID NO: 12, 32, 15, 20, and the FcB region;
or, amino acid sequence of the chain B consists of sequentially linked SEQ ID NO: 12, 32, 15, 21, and the FcB region;
or, amino acid sequence of the chain B consists of sequentially linked SEQ ID NO: 12, 32, 15, 22, and the FcB region;
or, amino acid sequence of the chain B consists of sequentially linked SEQ ID NO: 12, 32, 16, 17, and the FcB region;
or, amino acid sequence of the chain B consists of sequentially linked SEQ ID NO: 12, 32, 16, 18, and the FcB region;
or, amino acid sequence of the chain B consists of sequentially linked SEQ ID NO: 12, 32, 16, 19, and the FcB region;
or, amino acid sequence of the chain B consists of sequentially linked SEQ ID NO: 12, 32, 16, 20, and the FcB region;
or, amino acid sequence of the chain B consists of sequentially linked SEQ ID NO: 12, 32, 16, 21, and the FcB region;
or, amino acid sequence of the chain B consists of sequentially linked SEQ ID NO: 12, 32, 16, 22, and the FcB region;
when amino acid sequence of the FcA region is SEQ ID NO: 26, amino acid sequence of the FcB region is SEQ ID NO: 27; when amino acid sequence of the FcA region is SEQ ID NO: 27, amino acid sequence of the FcB region is SEQ ID NO: 26.

16. The bispecific recombinant protein according to claim 10, wherein
when the first functional binding region targets GPC3, and amino acid sequence of the chain 1 in the first arm consists of sequentially linked SEQ ID NO: 1, 31, and the FcA region, amino acid sequence of the chain 2 consists of sequentially linked SEQ ID NO: 2 and 32, and amino acid sequence of the second arm consists of sequentially linked any one of SEQ ID NOs: 17 to 22 and the FcB region; when amino acid sequence of the FcA region is SEQ ID NO: 26, amino acid sequence of the FcB region is SEQ ID NO: 27; or, when amino acid sequence of the FcA region is SEQ ID NO: 27, amino acid sequence of the FcB region is SEQ ID NO: 26;
when the first functional binding region targets PD-L1, and amino acid sequence of the chain 1 in the first arm consists of sequentially linked SEQ ID NO: 5, 31, and the FcA region, amino acid sequence of the chain 2 consists of sequentially linked SEQ ID NO: 6 and 32, and amino acid sequence of the second arm consists of sequentially linked any one of SEQ ID NOs: 17 to 22 and the FcB region; when amino acid sequence of the FcA region is SEQ ID NO: 26, amino acid sequence of the FcB region is SEQ ID NO: 27; or, when amino acid sequence of the FcA region is SEQ ID NO: 27, amino acid sequence of the FcB region is SEQ ID NO: 26; or, when amino acid sequence of the FcA region is SEQ ID NO: 29, amino acid sequence of the FcB region is SEQ ID NO: 30; or, when amino acid sequence of the FcA region is SEQ ID NO: 30, amino acid sequence of the FcB region is SEQ ID NO: 29;
when the first functional binding region targets BCMA, and amino acid sequence of the chain 1 in the first arm consists of sequentially linked SEQ ID NO: 7, 31, and the FcA region, amino acid sequence of the chain 2 consists of sequentially linked SEQ ID NO: 8 and 32; or, when amino acid sequence of the chain 1 in the first arm consists of sequentially linked SEQ ID NO: 9, 31, and the FcA region, amino acid sequence of the chain 2 in the first arm consists of sequentially linked SEQ ID NO: 10 and 32; amino acid sequence of the second arm consists of sequentially linked any one of SEQ ID NOs: 17 to 22 and the FcB region; when amino acid sequence of the FcA region is SEQ ID NO: 26, amino acid sequence of the FcB region is SEQ ID NO: 27; or, when amino acid sequence of the FcA region is SEQ ID NO: 27, amino acid sequence of the FcB region is SEQ ID NO: 26;
when the first functional binding region targets Trop-2, and amino acid sequence of the chain 1 in the first arm consists of sequentially linked SEQ ID NO: 11, 31, and the FcA region, amino acid sequence of the chain 2 consists of sequentially linked SEQ ID NO: 12 and 32, and amino acid sequence of the second arm consists of sequentially linked any one of SEQ ID NOs: 17 to 22 and the FcB region; when amino acid sequence of the FcA region is SEQ ID NO: 26, amino acid sequence of the FcB region is SEQ ID NO: 27; or, when amino acid sequence of the FcA region is SEQ ID NO: 27, amino acid sequence of the FcB region is SEQ ID NO: 26.

17. A nucleic acid molecule encoding the bispecific recombinant protein according to any one of claims 1 to 16.

18. A recombinant expression vector comprising the nucleic acid molecule according to claim 17.

19. A recombinant cell obtained by transforming a host cell with the expression vector according to claim 18.

20. A method for preparing the bispecific recombinant protein according to any one of claims 1 to 16, wherein the method includes the step of obtaining the bispecific recombinant protein by expression using the recombinant cell according to claim 19.

21. A medicament or pharmaceutical composition comprising the bispecific recombinant protein according to any one of claims 1 to 16;
preferably, the pharmaceutical composition further comprises a pharmaceutically acceptable carrier.

22. A use of the bispecific recombinant protein according to any one of claims 1 to 16 or the medicament or pharmaceutical composition according to claim 21 in the preparation of a medicament for the treatment, adjuvant therapy, or prevention of a disease;
preferably, the disease is selected from the group consisting of tumors, liver diseases, and viral infectious diseases;
more preferably, the disease is a GPC3, BCMA, PD-L1, Trop-2, 5T4, AGS-16, ALK1, ANG-2, B7-H3, B7-H4, c-fms, c-Met, CA6, CD123, CD19, CD20, CD22, CD24, EpCAM, CD30, CD32b, CD37, CD38, CD40, CD52, CD70, CD74, CD79b, CD98, CEA, CEACAM5, CLDN18.2, CLDN6, CS1, CXCR4, DLL-4, EGFR, EGFRvIII, EGP-1, ENPP3, EphA3, ETBR, FGFR2, FN, FR-α, GCC, GD2, GPNMB, HER2, HER3, HLA-DR, ICAM-1, IGF-1R, IL-3R, LIV-1, MSLN, MUC16, MUC1, NaPi2b, Nectin-4, Notch 2, Notch 1, PD-L2, PDGFR-α, PS, PSMA, SLTRK6, STEAP1, TEM1, VEGFR, CD25, CD27L, DKK-1, CSF-1R, MSB0010718C, CD138, Sig1ec15, or CD155-positive related disease.

23. The use according to claim 22, wherein the tumor is selected from the group consisting of breast cancer, intestinal cancer, pancreatic cancer, esophageal cancer, ovarian cancer, stomach cancer, prostate cancer, renal cancer, cervical cancer, myeloma, lymphoma, leukemia, thyroid cancer, uterine cancer, bladder cancer, neuroendocrine tumor, head and neck cancer, liver cancer, nasopharyngeal cancer, testicular cancer, lung cancer, melanoma, skin cancer, sarcoma, glioma, mesothelioma, and myelodysplastic syndrome;
preferably, the intestinal cancer includes colorectal cancer, the ovarian cancer includes yolk sac tumor, the neuroendocrine tumor includes Merkel cell carcinoma, the lung cancer includes small cell lung cancer and non-small cell lung cancer, the skin cancer includes basal cell skin cancer and squamous cell skin cancer, the sarcoma includes dermatofibrosarcoma protuberans, the glioma includes glioblastoma, and the uterine cancer includes endometrial cancer and uterine sarcoma.
